# EUROPEAN PATENT APPLICATION

(11) **EP 3 825 321 A1**
(43) Date of publication of application: **26.05.2021**
(21) Application number: 18926927.7
(22) Date of filing: 20.07.2018
(51) Int. Cl.: C07H 21/00, A61P 35/00

(54) **NUCLEIC ACID UNIT, POLYMERIC NUCLEIC ACID AND USE THEREOF**

(71) Applicant: Guangzhou Ribobio Co., Ltd., Guangzhou 510530 (CN)
(72) Inventor: ZHANG, Biliang, Guangzhou Development District Guangzhou, Guangdong 510663 (CN); YANG, Xiuqun, Guangzhou Development District Guangzhou, Guangdong 510663 (CN); SAMARSKY, Dmitry, Guangzhou Development District Guangzhou, Guangdong 510663 (CN)
(74) Representative: Novagraaf Technologies
(86) International application number: PCT/CN2018/096397
(87) International publication number: WO 2020/014948

(57) **Abstract**

The present application discloses a new-type nucleic acid unit for construction of a polymeric nucleic acid and the polymeric nucleic acid for interfering with target gene expression. In the present application, by means of the design and construction of the new-type nucleic acid unit and the self-assembled polymeric nucleic acid thereof, multiple target interference may be realized, wherein the same may be used for inhibiting multiple gene expressions in a signaling pathway of disease occurrence or development, or simultaneously inhibiting multiple disease target genes expression, possessing broad application prospects in multiple subject fields such as biology and chemistry. The polymeric nucleic acid may target multiple sequences simultaneously, wherein the sequences may be located in one or more genes. The advantages of the present application lie in: 1) a high RNAi performance; 2) good stability; 3) reduction of an off-target rate; 4) enhanced ability to be introduced into cells; and 5) a modular design.

SEQUENCE LISTING

<110> GUANGZHOU RIBOBIO CO.,LTD.

<120> NUCLEIC ACID UNIT, POLYMERIC NUCLEIC ACID AND APPLICATION THEREOF

<130> GNPLY18029

<160> 101

<170> PatentIn version 3.5

<210> 1
<211> 30bp
<212> RNA
<213> Artifical Sequence
<400> 1
agaugcucuu uccuccugca agguguauuu 30

<210> 2
<211> 30bp
<212> RNA
<213> Artifical Sequence
<400> 2
aaauacaccu ugacggugga ugaagaugua 30

<210> 3
<211> 30bp
<212> RNA
<213> Artifical Sequence
<400> 3
uacaucuuca ucuccaauuc ucuucggaaa 30

<210> 4
<211> 30bp
<212> RNA
<213> Artifical Sequence
<400> 4
uuuccgaaga gaccaaagga aagagcaucu 30

<210> 5
<211> 30bp
<212> RNA
<213> Artifical Sequence
<400> 5
uuuccgaaga gaccaaaguc uacgagaaag 30

<210> 6
<211> 16bp
<212> RNA
<213> Artifical Sequence
<400> 6
ggaggaaaga gcaucu 16

<210> 7
<211> 16bp
<212> RNA
<213> Artifical Sequence
<400> 7
cuuucucgua gacuuu 16

<210> 8

<211> 24bp
<212> RNA
<213> Artifical Sequence
<400> 8
cuuuggauug gacaccguca ggag                                          24

<210> 9
<211> 30bp
<212> RNA
<213> Artifical Sequence
<400> 9
agaugcucuu uccuccugca ugaaggugcu                                    30

<210> 10
<211> 30bp
<212> RNA
<213> Artifical Sequence
<400> 10
agcaccuuca uguugcguca agguguauuu                                    30

<210> 11
<211> 30bp
<212> RNA
<213> Artifical Sequence
<400> 11
uuuccgaaga gaccaaagcg uguaaucaag                                    30

<210> 12
<211> 30bp
<212> RNA
<213> Artifical Sequence
<400> 12
cuugauuaca cgauggaaga aagagcaucu                                    30


<210> 13
<211> 30bp
<212> RNA
<213> Artifical Sequence
<400> 13
cuugauuaca cgauggaauc uacgagaaag                                    30

<210> 14
<211> 16bp
<212> RNA
<213> Artifical Sequence
<400> 14
cuuucucgua gauucc                                                   16

<210> 15
<211> 30bp
<212> RNA
<213> Artifical Sequence
<400> 15
uguguagcca uugaucuugc aucaugaaga                                    30

<210> 16
<211> 30bp
<212> RNA
<213> Artifical Sequence
<400> 16
ucuucaugau gcucuugaau accaccaaga                                    30

<210> 17
<211> 30bp
<212> RNA
<213> Artifical Sequence
<400> 17
ucuugguggu aucugugcuc gucaccggau                                        30

<210> 18
<211> 30bp
<212> RNA
<213> Artifical Sequence
<400> 18
auccggugac gaucgucuaa uggcuacaca                                        30

<210> 19
<211> 30bp
<212> RNA
<213> Artifical Sequence
<400> 19
auccggugac gaucgucuac acaucgguaa                                        30

<210> 20
<211> 16bp
<212> RNA
<213> Artifical Sequence
<400> 20
gaucaauggc uacaca                                                       16

<210> 21
<211> 16bp
<212> RNA
<213> Artifical Sequence
<400> 21
uuaccgaugu guagac                                                       16

<210> 22
<211> 24bp
<212> RNA
<213> Artifical Sequence
<400> 22
agacgagcac agucaagaaa gauc                                              24


<210> 23
<211> 30bp
<212> RNA
<213> Artifical Sequence
<400> 23
auccggugac gaucgucuuc aggagaugaa                                        30

<210> 24
<211> 30bp
<212> RNA
<213> Artifical Sequence
<400> 24
uucaucuccu gaaaggagau cagcuccuaa                                        30

<210> 25
<211> 30bp
<212> RNA
<213> Artifical Sequence
<400> 25
uuaggagcug aucugacuaa uggcuacaca                                        30

<210> 26
<211> 30bp
<212> RNA
<213> Artifical Sequence
<400> 26
uuaggagcug aucugacuac acaucgguaa                                        30

<210> 27
<211> 16bp
<212> RNA
<213> Artifical Sequence
<400> 27
uuaccgaugu guaguc                                                       16

<210> 28
<211> 30bp
<212> RNA
<213> Artifical Sequence
<400> 28
agaagaaaca cugggccaac ugcugaucuu                                        30

<210> 29
<211> 30bp
<212> RNA
<213> Artifical Sequence
<400> 29
aagaucagca gucucauuug gucaggucuu                                        30

<210> 30
<211> 30bp
<212> RNA
<213> Artifical Sequence
<400> 30
aagaccugac cagugguauu ugacucugau                                        30

<210> 31
<211> 30bp
<212> RNA
<213> Artifical Sequence
<400> 31
aucagaguca aagagcuuag uguuucuucu                                        30

<210> 32
<211> 30bp
<212> RNA
<213> Artifical Sequence
<400> 32
ucaaguugcu ggucaucagg agguugcuaa                                        30


<210> 33
<211> 30bp
<212> RNA
<213> Artifical Sequence
<400> 33
uuagcaaccu ccugauuaag uguuucuucu                                        30

<210> 34
<211> 30bp
<212> RNA
<213> Artifical Sequence
<400> 34

aucagaguca aagagcuucc agcaacuuga 30

<210> 35
<211> 30bp
<212> RNA
<213> Artifical Sequence
<400> 35
uacuuucuuc auuuccaccu guuccaaaaa 30

<210> 36
<211> 30bp
<212> RNA
<213> Artifical Sequence
<400> 36
uuuuuggaac agucuuucug agaccuucaa 30

<210> 37
<211> 30bp
<212> RNA
<213> Artifical Sequence
<400> 37
uugaaggucu cauaugucca ugcagauuau 30

<210> 38
<211> 30bp
<212> RNA
<213> Artifical Sequence
<400> 38
auaaucugca uggugaugau gaagaaagua 30

<210> 39
<211> 16bp
<212> RNA
<213> Artifical Sequence
<400> 39
ggaaaugaag aaagua 16

<210> 40
<211> 16bp
<212> RNA
<213> Artifical Sequence
<400> 40
uacuuucuuc aucauc 16

<210> 41
<211> 24bp
<212> RNA
<213> Artifical Sequence
<400> 41
caucacgaca uagaaagagu ggaa 24

<210> 42
<211> 30bp
<212> RNA
<213> Artifical Sequence
<400> 42
agcagaaagu ucaugguuuc uugggugcau 30

<210> 43
<211> 30bp
<212> RNA
<213> Artifical Sequence

<400> 43
augcacccaa gacagcagag aucgaguaca                                    30

<210> 44
<211> 30bp
<212> RNA
<213> Artifical Sequence
<400> 44
uguacucgau cucaucaggg uggacaucuu                                    30

<210> 45
<211> 30bp
<212> RNA
<213> Artifical Sequence
<400> 45
aagaugucca ccagggucau gcggaucaaa                                    30

<210> 46
<211> 30bp
<212> RNA
<213> Artifical Sequence
<400> 46
uuugauccgc auaaucuggg ccagcacaua                                    30

<210> 47
<211> 30bp
<212> RNA
<213> Artifical Sequence
<400> 47
uaugugcugg ccuuggugga acuuucugcu                                    30

<210> 48
<211> 29bp
<212> RNA
<213> Artifical Sequence
<400> 48
agcagaaagu ucaugguucu ugggugcau                                     29

<210> 49
<211> 29bp
<212> RNA
<213> Artifical Sequence
<400> 49
augcacccaa gacagcaaga ucgaguaca                                     29

<210> 50
<211> 29bp
<212> RNA
<213> Artifical Sequence
<400> 50
uguacucgau cucaucaggu ggacaucuu                                     29

<210> 51
<211> 29bp
<212> RNA
<213> Artifical Sequence
<400> 51
aagaugucca ccaggguaug cggaucaaa                                     29

<210> 52
<211> 29bp
<212> RNA
<213> Artifical Sequence

<400> 52
uuugauccgc auaaucuggc cagcacaua                                    29

<210> 53
<211> 29bp
<212> RNA
<213> Artifical Sequence
<400> 53
uaugugcugg ccuuggugaa cuuucugcu                                    29

<210> 54
<211> 27bp
<212> RNA
<213> Artifical Sequence
<400> 54
agcagaaagu ucaugucuug ggugcau                                      27

<210> 55
<211> 27bp
<212> RNA
<213> Artifical Sequence
<400> 55
augcacccaa gacagagauc gaguaca                                      27

<210> 56
<211> 27bp
<212> RNA
<213> Artifical Sequence
<400> 56
uguacucgau cucauggugg acaucuu                                      27

<210> 57
<211> 27bp
<212> RNA
<213> Artifical Sequence
<400> 57
aagaugucca ccaggaugcg gaucaaa                                      27

<210> 58
<211> 27bp
<212> RNA
<213> Artifical Sequence
<400> 58
uuugauccgc auaauggcca gcacaua                                      27

<210> 59
<211> 27bp
<212> RNA
<213> Artifical Sequence
<400> 59
uaugugcugg ccuuggaacu uucugcu                                      27

<210> 60
<211> 28bp
<212> RNA
<213> Artifical Sequence
<400> 60
agcagaaagu ucauggucuu gggugcau                                     28

<210> 61
<211> 28bp
<212> RNA

<213> Artifical Sequence
<400> 61
augcacccaa gacagcagau cgaguaca                                          28

<210> 62
<211> 28bp
<212> RNA
<213> Artifical Sequence
<400> 62
uguacucgau cucaucagug gacaucuu                                          28


<210> 63
<211> 28bp
<212> RNA
<213> Artifical Sequence
<400> 63
aagaugucca ccaggguugc ggaucaaa                                          28

<210> 64
<211> 28bp
<212> RNA
<213> Artifical Sequence
<400> 64
uuugauccgc auaaucugcc agcacaua                                          28

<210> 65
<211> 28bp
<212> RNA
<213> Artifical Sequence
<400> 65
uaugugcugg ccuugguaac uuucugcu                                          28

<210> 66
<211> 26bp
<212> RNA
<213> Artifical Sequence
<400> 66
agcagaaagu ucaugguugg gugcau                                            26

<210> 67
<211> 26bp
<212> RNA
<213> Artifical Sequence
<400> 67
augcacccaa gacagcaucg aguaca                                            26

<210> 68
<211> 26bp
<212> RNA
<213> Artifical Sequence
<400> 68
uguacucgau cucaucugga caucuu                                            26

<210> 69
<211> 26bp
<212> RNA
<213> Artifical Sequence
<400> 69
aagaugucca ccagggcgg aucaaa                                             26

<210> 70
<211> 26bp

<212> RNA
<213> Artifical Sequence
<400> 70
uuugauccgc auaaucccag cacaua                                          26

<210> 71
<211> 26bp
<212> RNA
<213> Artifical Sequence
<400> 71
uaugugcugg ccuuggacuu ucugcu                                          26

<210> 72
<211> 30bp
<212> RNA
<213> Artifical Sequence
<400> 72
uguggaauca acccacaguu ugcgugugga                                      30


<210> 73
<211> 30bp
<212> RNA
<213> Artifical Sequence
<400> 73
uccacacgca aauuuccuac agaaacacuu                                      30

<210> 74
<211> 30bp
<212> RNA
<213> Artifical Sequence
<400> 74
aaguguuucu gucauccaac uacaugugua                                      30

<210> 75
<211> 30bp
<212> RNA
<213> Artifical Sequence
<400> 75
uacacaugua guuguaguug guaaucuacu                                      30

<210> 76
<211> 30bp
<212> RNA
<213> Artifical Sequence
<400> 76
aguagauuac cacuggaguc uccgcaagaa                                      30

<210> 77
<211> 30bp
<212> RNA
<213> Artifical Sequence
<400> 77
uucuugcgga gauucucugu ugauuccaca                                      30

<210> 78
<211> 29bp
<212> RNA
<213> Artifical Sequence
<400> 78
uguggaauca acccacauuu gcgugugga                                       29

<210> 79

<211> 29bp
<212> RNA
<213> Artifical Sequence
<400> 79
uccacacgca aauuuccaca gaaacacuu 29

<210> 80
<211> 29bp
<212> RNA
<213> Artifical Sequence
<400> 80
aaguguuucu gucauccacu acaugugua 29

<210> 81
<211> 29bp
<212> RNA
<213> Artifical Sequence
<400> 81
uacacaugua guuguagugg uaaucuacu 29

<210> 82
<211> 29bp
<212> RNA
<213> Artifical Sequence
<400> 82
aguagauuac cacuggaucu ccgcaagaa 29

<210> 83
<211> 29bp
<212> RNA
<213> Artifical Sequence
<400> 83
uucuugcgga gauucucguu gauuccaca 29

<210> 84
<211> 27bp
<212> RNA
<213> Artifical Sequence
<400> 84
uguggaauca acccauuugc gugugga 27

<210> 85
<211> 27bp
<212> RNA
<213> Artifical Sequence
<400> 85
uccacacgca aauuuacaga aacacuu 27

<210> 86
<211> 27bp
<212> RNA
<213> Artifical Sequence
<400> 86
aaguguuucu gucauacuac augugua 27

<210> 87
<211> 27bp
<212> RNA
<213> Artifical Sequence
<400> 87
uacacaugua guuguuggua aucuacu 27

<210> 88
<211> 27bp
<212> RNA
<213> Artifical Sequence
<400> 88
aguagauuac cacugucucc gcaagaa                                          27

<210> 89
<211> 27bp
<212> RNA
<213> Artifical Sequence
<400> 89
uucuugcgga gauucguuga uuccaca                                          27

<210> 90
<211> 28bp
<212> RNA
<213> Artifical Sequence
<400> 90
uguggaauca acccacauug cgugugga                                         28

<210> 91
<211> 28bp
<212> RNA
<213> Artifical Sequence
<400> 91
uccacacgca aauuucccag aaacacuu                                         28

<210> 92
<211> 28bp
<212> RNA
<213> Artifical Sequence
<400> 92
aaguguuucu gucaucccua caugugua                                         28


<210> 93
<211> 28bp
<212> RNA
<213> Artifical Sequence
<400> 93
uacacaugua guuguagggu aaucuacu                                         28

<210> 94
<211> 28bp
<212> RNA
<213> Artifical Sequence
<400> 94
aguagauuac cacuggacuc cgcaagaa                                         28

<210> 95
<211> 28bp
<212> RNA
<213> Artifical Sequence
<400> 95
uucuugcgga gauucucuug auuccaca                                         28

<210> 96
<211> 26bp
<212> RNA
<213> Artifical Sequence
<400> 96
uguggaauca acccacugcg ugugga                                           26

<210> 97
<211> 26bp
<212> RNA
<213> Artifical Sequence
<400> 97
uccacacgca aauuucagaa acacuu                                          26

<210> 98
<211> 26bp
<212> RNA
<213> Artifical Sequence
<400> 98
aaguguuucu gucaucuaca ugugua                                          26

<210> 99
<211> 26bp
<212> RNA
<213> Artifical Sequence
<400> 99
uacacaugua guuguaguaa ucuacu                                          26

<210> 100
<211> 26bp
<212> RNA
<213> Artifical Sequence
<400> 100
aguagauuac cacugguccg caagaa                                          26

<210> 101
<211> 25bp
<212> RNA
<213> Artifical Sequence
<400> 101
ucuugcggag auucuugauu ccaca                                           25

## Description

### Technical Field

The present disclosure belongs to the technical field of nucleic acids, and particularly relates to a nucleic acid unit in new-type structure and a self-assembled polymeric nucleic acid thereof, the polymeric nucleic acid may interfere with expressions of one or more target genes.

### Background

A complementary oligonucleotide (CON) technology makes an oligonucleotide molecule synthesized artificially bind to a target molecule through sequence complementation, and change the biological characteristics of the target molecule. The CON technology includes two types of a RNA interference (RNAi) technology and an antisense oligonucleotide (ASO) technology, it is widely used in functional genomics research at present, and expected to become the third greatest treatment means in addition to small molecule compounds and biological agents. For example, mipomersen for treating homozygous familial hypercholesterolemia (FoFH), it is a synthetic phosphorothioate oligonucleotide developed by Genzyme, through complementary pairing with a coding region of an Apo B-100 protein mRNA, translation and synthesis of an Apo B-100 protein (main apolipoproteins of LDL and VLDL) are inhibited, thereby LDL-C, TC, and Non-HDL-C levels of a FoFH patient are effectively reduced. Although the CON technology achieves a certain success, this technology still needs to be improved. For example, a traditional RNAi reagent has the following disadvantages: 1) complicated synthetic steps and relatively high production cost; 2) high sensitivity to endonuclease and exonuclease, and low stability; 3) inhibition efficiency is not high enough, it may not be guaranteed that a single molecule may inhibit an expression of a target gene necessarily; 4) side effects caused by non-specific activity, the non-specific effect is mainly derived from a sense strand; and 5) it is difficult to be introduced into a cell, especially animals.

A nanotechnology researches properties and applications of a substance in diameter within a nano range of 1-100 nm. The characteristics, such as a small size effect, a surface effect and a high diffusivity, of the substance in nanostructure open up a new field for scientific research and technical application. The oligonucleotide is flexible in structure, a RNA structure for example, it may form a nanostructure by self-assembly. A nucleic acid nanotechnology represented by DNA and RNA is a new direction of development in the field of nanotechnologies.

### Summary

A technical problem to be solved by the present disclosure is how to inhibit or reduce or interfere with an expression of a target gene.

In order to solve the above technical problem, the present disclosure firstly provides a polymeric nucleic acid molecule for inhibiting or reducing or interfering the expression of the target gene.

The polymeric nucleic acid molecule for inhibiting or reducing or interfering with the expression of the target gene provided by the present disclosure is formed by n X-type nucleic acid molecules.

Each of the X-type nucleic acid molecules is formed by a targeting segment I, a targeting segment II and a linker segment III successively from a 5'-end. The targeting segment I of each of the X-type nucleic acid molecules is complementary-paired with the linker segment III of an adjacent X-type nucleic acid molecule thereof. Specifically, the n X-type nucleic acid molecules are respectively named as an X1 unit, an X2 unit, an X3 unit, and so on, an Xn-1 unit, and an Xn unit; the linker segment III of the X1 unit is complementary-paired (complete complementary) with the targeting segment I of the X2 unit, the linker segment III of the X2 unit is complementary-paired (complete complementary) with the targeting segment I of the X3 unit, and so on, the linker segment III of the Xn-1 unit is complementary-paired (complete complementary) with the targeting segment I of the Xn unit, and the linker segment III of the Xn unit is complementary-paired (complete complementary) with the targeting segment I of the X1 unit. The targeting segment I of each of the X-type nucleic acid molecules is not complementary to other X-type nucleic acid molecule sequences.

The targeting segment I and the targeting segment II of each of the polymeric nucleic acid molecules are complementary-paired with a target gene; each of the polymeric nucleic acid molecules is combined with a specific sequence of the target gene through the targeting segment I and the targeting segment II, so as to inhibit or reduce or interfere with the expression of the target gene. In certain specific cases, a region complementary to a target gene sequence may be prolonged to a partial sequence of the linker segment III. The targeting segment I and the targeting segment II in the same X-type nucleic acid molecule may be the same, or may be different. The targeting segment I or the targeting segment II in two different X-type nucleic acid molecules may be the same, or may be different.

A length of each of the X-type nucleic acid molecules is the same (a structure is the same too).

The n is an integer greater than or equal to 3.

In a specific embodiment of the present disclosure, the n X-type nucleic acid molecules are successively connected end to end (achieved by complementation of the linker segment III of the previous X-type nucleic acid molecule and the targeting segment I of the next adjacent X-type nucleic acid molecule), finally a polymeric nucleic acid molecule with a cyclic secondary structure is formed.

In the above polymeric nucleic acid molecule, the n X-type nucleic acid molecules may also form a polymeric nucleic acid molecule with a linear structure, the polymeric nucleic acid molecule with the linear structure further includes a H-type nucleic acid molecule, and the H-type nucleic acid molecule is formed by a H1-type nucleic acid molecule and a Hn-type nucleic acid molecule.

n X-type nucleic acid molecules are respectively named as an X1 unit, an X2 unit, an X3 unit, and so on, an Xn-1 unit, and an Xn unit; the linker segment III of the X1 unit is complementary-paired with the targeting segment I of the X2 unit, the linker segment III of the X2 unit is complementary-paired with the targeting segment I of the X3 unit, and so on, the linker segment III of the Xn-1 unit is complementary-paired with the targeting segment I of the Xn unit; the H1-type nucleic acid molecule is complementary-paired with the targeting segment I of the X1 unit; and the Hn-type nucleic acid molecule is complementary-paired with the linker segment III of the Xn unit.

Further, 5'-end or 3'-end of the H-type nucleic acid molecule further includes a Hy segment; and the H-type nucleic acid molecule is formed by an Hx segment and the Hy segment.

A complementary region of the H1-type nucleic acid molecule and the X1 unit may be extended to partial or all sequences of the targeting segment II of the X1 unit; and a complementary region of the Hn-type nucleic acid molecule and the Xn unit may be extended to partial or all sequences of the targeting segment II of the Xn unit.

The Hx segment of the H1-type nucleic acid molecule is complementary-paired with the targeting segment I of the X1 unit; the Hx segment of the Hn-type nucleic acid molecule is complementary-paired with the linker segment III of the Xn unit; and the Hy segment is complementary-paired with one, two or more continuous nucleic acid molecules of the targeting segment II of the X1 unit or the Xn unit from 5'-end or 3'-end.

In the above polymeric nucleic acid molecules, the polymeric nucleic acid molecule with the cyclic structure further includes a C-type nucleic acid molecule; the C-type nucleic acid molecule is formed by n segments connected successively, the n segments are respectively reverse complementary sequences of the targeting segments II in the n X-type nucleic acid molecules. Specifically, the C-type nucleic acid molecule is formed by a capping-end segment 1, a capping-end segment 2, a capping-end segment 3, and so on, a capping-end segment Cn-1, and a capping-end segment n successively from a 3'-end; and n X-type nucleic acid molecules are respectively named as an X1 unit, an X2 unit, an X3 unit, and so on, an Xn-1 unit, and an Xn unit;
the linker segment III of the X1 unit is complementary-paired with the targeting segment I of the X2 unit, the linker segment III of the X2 unit is complementary-paired with the targeting segment I of the X3 unit, and so on, the linker segment III of the Xn-1 unit is complementary-paired with the targeting segment I of the Xn unit, and the linker segment III of the Xn unit is complementary-paired with the targeting segment I of the X1 unit; the capping-end segment 1 is complementary-paired with the targeting segment II of the X1 unit, the capping-end segment 2 is complementary-paired with the targeting segment II of the X2 unit, and so on, the capping-end segment Cn-1 is complementary-paired with the targeting segment II of the Xn-1 unit, and the capping-end segment n is complementary-paired with the targeting segment II of the Xn unit.

In the above polymeric nucleic acid molecules, the nucleic acid molecule (the X-type nucleic acid molecule, the H-type nucleic acid molecule or the C-type nucleic acid molecule) may be a DNA or a RNA or an oligonucleotide formed by the DNA and the RNA.

Further, the nucleic acid molecule (the X-type nucleic acid molecule, the H-type nucleic acid molecule or the C-type nucleic acid molecule) is a single-stranded RNA molecule.

In the above polymeric nucleic acid molecules, a length of the X-type nucleic acid molecule is 15-50 nt, preferably 24-36 nt;
a length of the targeting segment I is 5-24 nt;
a length of the targeting segment II is 1-20 nt;
a length of the linker segment III is 5-24 nt;
a sum of the lengths of the targeting segment I and the targeting segment II is 14-16 nt at least; and
a length of the Hy segment is 2-6 nt or longer.

Further, the length of the X-type nucleic acid molecule is 24-36 nt.

In the above polymeric nucleic acid molecules, the polymeric nucleic acid molecule at least includes a modified nucleotide.

Further, the modification is phosphoric acid backbone modification, base modification and/or ribose modification. The ribose modification is that a ribose 2-site hydroxyl group is substituted by a halogen group or an O-alkyl group. The alkyl is a methyl, an ethyl, a propyl or a methylethyl.

Furthermore, the ribose 2-site hydroxyl groups of 5-9 continuous nucleotides, from the first nucleotide at the 3'-end, of the linker segment III of the X-type nucleic acid molecule are substituted by the halogen group or the O-alkyl group;
the ribose 2-site hydroxyl groups of 5-9 continuous nucleotides, from the first nucleotide at the 3'-end, of the Hx segment of the H-type nucleic acid molecule are substituted by the halogen group or the O-alkyl group;
the ribose 2-site hydroxyl groups of 8-30 continuous nucleotides, from the first nucleotide at the 3'-end, of the H-type nucleic acid molecule are substituted by the halogen group or the O-alkyl group; preferably, the ribose 2-site hydroxyl groups of 14-18 continuous nucleotides, from the first nucleotide at the 3'-end, of the H-type nucleic acid molecule are substituted by the halogen group or the O-alkyl group; and
the ribose 2-site hydroxyl groups of 2-6 continuous nucleotides, from the first nucleotide at the 5'-end, of each capping-end segment in the C-type nucleic acid molecule are substituted by the halogen group or the O-alkyl group.

In the above polymeric nucleic acid molecules, the n is 3 or 4 or 5 or 6 or 7 or 8.
Further, the n is 4 or 5 or 6.

In the above polymeric nucleic acid molecules, the number of the target genes is one or two or more, and the number of the target genes does not exceed n; and each X-type nucleic acid molecule corresponds to one target gene, or multiple X-type nucleic acid molecules correspond to different regions of the same target gene.

Further, the number of the target genes is 1 or 4 or 6.

The target gene is at least one of the following genes: *PPIB, p65, BIRC5, CTNNB, COPS5, CLU, EIF4E, HIF1A, TP53, VEGFA* and *SOD1.*

Furthermore, the polymeric nucleic acid molecule for interfering with an expression of the target gene *PPIB* is the following a1)-a5):
a1) is formed by single-stranded RNA molecules shown in SEQ ID NO:1, SEQ ID NO:2, SEQ ID NO:3 and SEQ ID NO:4;
a2) is formed by single-stranded RNA molecules shown in SEQ ID NO:1, SEQ ID NO:2, SEQ ID NO:3, SEQ ID NO:4, SEQ ID NO:20 and SEQ ID NO:21;
a3) is formed by single-stranded RNA molecules shown in SEQ ID NO:1, SEQ ID NO:2, SEQ ID NO:3, SEQ ID NO:4 and SEQ ID NO:8;
a4) is formed by single-stranded RNA molecules shown in SEQ ID NO:2, SEQ ID NO:3, SEQ ID NO:9, SEQ ID N0:10, SEQ ID NO:11 and SEQ ID NO:12;
a5) is formed by single-stranded RNA molecules shown in SEQ ID NO:2, SEQ ID NO:3, SEQ ID NO:6, SEQ ID NO:9, SEQ ID N0:10, SEQ ID NO:11, SEQ ID NO:13 and SEQ ID NO:14;
the polymeric nucleic acid molecule for interfering with an expression of the target gene *P65* is the following b1)-b5):
b1) is formed by single-stranded RNA molecules shown in SEQ ID NO:15, SEQ ID NO:16, SEQ ID NO:17 and SEQ ID NO:18;
b2) is formed by single-stranded RNA molecules shown in SEQ ID NO:6, SEQ ID NO:7, SEQ ID NO:15, SEQ ID NO:16, SEQ ID NO:17 and SEQ ID NO:19;
b3) is formed by single-stranded RNA molecules shown in SEQ ID NO:15, SEQ ID NO:16, SEQ ID NO:17, SEQ ID NO:18 and SEQ ID NO:22;
b4) is formed by single-stranded RNA molecules shown in SEQ ID NO:15, SEQ ID NO:16, SEQ ID NO:17, SEQ ID NO:23, SEQ ID NO:24 and SEQ ID NO:25;
b5) is formed by single-stranded RNA molecules shown in SEQ ID NO:15, SEQ ID NO:16, SEQ ID NO:17, SEQ ID NO:20, SEQ ID NO:23, SEQ ID NO:24, SEQ ID NO:26 and SEQ ID NO:27;
the polymeric nucleic acid molecule for simultaneously interfering with expressions of the target genes *BIRC5, CTNNB, COPS5* and *CLU* is formed by single-stranded RNA molecules shown in SEQ ID NO:28, SEQ ID NO:29, SEQ ID NO:30 and SEQ ID NO:31;
the polymeric nucleic acid molecule for simultaneously interfering expressions of the target genes *BIRC5, CTNNB, COPS5, CLU, EIF4E* and *HIF1A* is formed by single-stranded RNA molecules shown in SEQ ID NO:28, SEQ ID NO:29, SEQ ID NO:30, SEQ ID NO:32, SEQ ID NO:33 and SEQ ID NO:34;
the polymeric nucleic acid molecule for simultaneously interfering with expressions of the target genes *SOD1, PPIB, P65* and *VEGFA* is the following c1)-c3):
c1) is formed by single-stranded RNA molecules shown in SEQ ID NO:35, SEQ ID NO:36, SEQ ID NO:37 and SEQ ID NO:38;
c2) is formed by single-stranded RNA molecules shown in SEQ ID NO:35, SEQ ID NO:36, SEQ ID NO:37, SEQ ID NO:38, SEQ ID NO:39 and SEQ ID NO:40;
c3) is formed by single-stranded RNA molecules shown in SEQ ID NO:35, SEQ ID NO:36, SEQ ID NO:37, SEQ ID NO:38 and SEQ ID NO:41;
the polymeric nucleic acid molecule for interfering with an expression of the target gene *VEGFA* is the following d1)-d11):
d1) is formed by single-stranded RNA molecules shown in SEQ ID NO:42, SEQ ID NO:43, SEQ ID NO:44, SEQ ID NO:45, sequence46 and SEQ ID NO:47;
d2) is formed by single-stranded RNA molecules shown in SEQ ID NO:48, SEQ ID NO:49, SEQ ID NO:50, SEQ ID NO:51, SEQ ID NO:52 and SEQ ID NO:53;
d3) is formed by single-stranded RNA molecules shown in SEQ ID NO:54, SEQ ID NO:55, SEQ ID NO:56, SEQ ID NO:57, SEQ ID NO:58 and SEQ ID NO:59;
d4) is formed by single-stranded RNA molecules shown in SEQ ID NO:42, SEQ ID NO:43, SEQ ID NO:45, SEQ ID NO:46 and SEQ ID NO:47;
d5) is formed by single-stranded RNA molecules shown in SEQ ID NO:48, SEQ ID NO:49, SEQ ID NO:51, SEQ ID NO:52 and SEQ ID NO:53;
d6) is formed by single-stranded RNA molecules shown in SEQ ID NO:54, SEQ ID NO:55, SEQ ID NO:57, SEQ ID NO:58 and SEQ ID NO:59;
d7) is formed by single-stranded RNA molecules shown in SEQ ID NO:42, SEQ ID NO:45, SEQ ID NO:46 and SEQ ID NO:47;
d8) is formed by single-stranded RNA molecules shown in SEQ ID NO:48, SEQ ID NO:51, SEQ ID NO:52 and SEQ ID NO:53;
d9) is formed by single-stranded RNA molecules shown in SEQ ID NO:54, SEQ ID NO:57, SEQ ID NO:58 and SEQ ID NO:59;
d10) is formed by single-stranded RNA molecules shown in SEQ ID NO:60, SEQ ID NO:61, SEQ ID NO:62, SEQ ID NO:63, SEQ ID NO:64 and SEQ ID NO:65;
d11) is formed by single-stranded RNA molecules shown in SEQ ID NO:66, SEQ ID NO:67, SEQ ID NO:68, SEQ ID NO:69, SEQ ID NO:70 and SEQ ID NO:71;
the polymeric nucleic acid molecule for interfering with an expression of the target gene *TP53* is the following e1)-e11):
e1) is formed by single-stranded RNA molecules shown in SEQ ID NO:72, SEQ ID NO:73, SEQ ID NO:74, SEQ ID NO:75, SEQ ID NO:76 and SEQ ID NO:77;
e2) is formed by single-stranded RNA molecules shown in SEQ ID NO:78, SEQ ID NO:79, SEQ ID NO:80, SEQ ID NO:81, SEQ ID NO:82 and SEQ ID NO:83;
e3) is formed by single-stranded RNA molecules shown in SEQ ID NO:84, SEQ ID NO:85, SEQ ID NO:86, SEQ ID NO:87, SEQ ID NO:88 and SEQ ID NO:89;
e4) is formed by single-stranded RNA molecules shown in SEQ ID NO:73, SEQ ID NO:74, SEQ ID NO:75, SEQ ID NO:76 and SEQ ID NO:77;
e5) is formed by single-stranded RNA molecules shown in SEQ ID NO:79, SEQ ID NO:80, SEQ ID NO:81, SEQ ID NO:82 and SEQ ID NO:83;
e6) is formed by single-stranded RNA molecules shown in SEQ ID NO:54, SEQ ID NO:55, SEQ ID NO:57, SEQ ID NO:58 and SEQ ID NO:59;
e7) is formed by single-stranded RNA molecules shown in SEQ ID NO:74, SEQ ID NO:75, SEQ ID NO:76 and SEQ ID NO:77;
e8) is formed by single-stranded RNA molecules shown in SEQ ID NO:80, SEQ ID NO:81, SEQ ID NO:82 and SEQ ID NO:83;
e9) is formed by single-stranded RNA molecules shown in SEQ ID NO:86, SEQ ID NO:87, SEQ ID NO:88 and SEQ ID NO:89;
e10) is formed by single-stranded RNA molecules shown in SEQ ID NO:90, SEQ ID NO:91, SEQ ID NO:92, SEQ ID NO:93, SEQ ID NO:94 and SEQ ID NO:95; and
e11) is formed by single-stranded RNA molecules shown in SEQ ID NO:96, SEQ ID NO:97, SEQ ID NO:98, SEQ ID NO:99, SEQ ID NO:100 and SEQ ID NO:101.

In order to solve the above technical problem, the present disclosure further provides a derivative of the above polymeric nucleic acid molecule.

The derivative of the above polymeric nucleic acid molecule provided by the present disclosure is any one of the following (m1)-(m5):
(m1) the above polymeric nucleic acid molecule is deleted or added one or more nucleotides, to obtain a derivative of the polymeric nucleic acid molecule having the same function as the polymeric nucleic acid molecule;
(m2) the above polymeric nucleic acid molecule is performed nucleotide substitution or modification, to obtain a derivative of the polymeric nucleic acid molecule having the same function as the polymeric nucleic acid molecule;
(m3) a backbone of the above polymeric nucleic acid molecule is transformed into a phosphorothioate backbone, to obtain a derivative of the polymeric nucleic acid molecule having the same function as the polymeric nucleic acid molecule;
(m4) a peptide nucleic acid, a locked nucleic acid or an unlocked nucleic acid coded by the above polymeric nucleic acid molecule is used, to obtain a derivative of the polymeric nucleic acid molecule having the same function as the polymeric nucleic acid molecule; and
(m5) one end or middle of the above polymeric nucleic acid molecule is linked with a signal molecule and/or an active molecule and/or a functional group, to obtain a derivative of the polymeric nucleic acid molecule having the same function as the polymeric nucleic acid molecule.

In order to solve the above technical problem, the present disclosure further provides a preparation method for the above polymeric nucleic acid molecule.

The preparation method for the above polymeric nucleic acid molecule provided by the present disclosure includes the following steps:
M1) the above X-type nucleic acid molecule and/or H-type nucleic acid molecule and/or C-type nucleic acid molecule is synthesized; and
M2) the X-type nucleic acid molecule and/or the H-type nucleic acid molecule and/or the C-type nucleic acid molecule is annealed, to obtain the polymeric nucleic acid.

In the above method, a reaction system for annealing is a system obtained by uniformly mixing each single-stranded RNA molecule in equal molar weight, RNA annealing buffer and water. A reaction condition for annealing is that a temperature is 90 DEG C in a polymerase chain reaction (PCR) meter, it is adequately denaturized in 2 min, and then the temperature in the PCR meter is reduced to 25 DEG C so that it is annealed.

While n is 4, the annealing system is as follows (a total volume is 100µL): 20µL of each single-stranded RNA molecule solution (the concentration is 100µM), 15µL of the RNA annealing buffer (5X), and 5µL of the DEPC water.

While n is 5, the annealing system is as follows (the total volume is 150µL): 20µL of each single-stranded RNA molecule solution (the concentration is 150µM), 30µL of the RNA annealing buffer (5X), and 20µL of the DEPC water.

While n is 6, the annealing system is as follows (the total volume is 200µL): 20µL of each single-stranded RNA molecule solution (the concentration is 200µM), 40µL of the RNA annealing buffer (5X), and 40µL of the DEPC water.

In order to solve the above technical problem, the present disclosure further provides a new application of the above polymeric nucleic acid molecule or derivative.

The present disclosure provides the application of the above polymeric nucleic acid molecule or derivative in the following A1) or A2):
A1) a target gene expression level in a cell is controlled; and
A2) a product for preventing and/or relieving and/or treating a disease caused by the target gene expression is prepared.

In the above application, the control is inhibition or reduction or interference.

In the above application, the cell is a tumor cell.

In the above application, the target gene is a disease-related gene; the disease-related gene is a tumor-related gene specifically; and the tumor-related gene is specifically at least one of the following genes: *PPIB*, *p65*, *BIRC5*, *CTNNB*, *COPS5*, *CLU, EIF4E, HIF1A, TP53, VEGFA* and *SOD1.*

In order to solve the above technical problem, the present disclosure further provides a reagent or a kit or a drug for inhibiting or reducing or interfering with a target gene expression level in a cell.

The reagent or the kit or the drug for inhibiting or reducing or interfering with the target gene expression level in the cell provided by the present disclosure includes the above polymeric nucleic acid molecule or the above derivative.

In order to solve the above technical problem, the present disclosure finally provides a method for inhibiting or reducing or interfering with a target gene expression level in a cell.

The method for inhibiting or reducing or interfering the target gene expression level in the cell provided by the present disclosure includes the following steps: the above polymeric nucleic acid molecule or derivative is introduced into the cell, and the expression level of the target gene in the cell is inhibited or reduced.

In the above method, the introduction method is that the polymeric nucleic acid molecule, a transfection reagent and buffer are added to a cell culture medium after being uniformly mixed, to obtain a reaction system, herein the final concentration of the polymeric nucleic acid molecule in the reaction system is 1-300 nM.

In the above method, the cell is a tumor cell.

In the above method, the target gene is a disease-related gene; the disease-related gene is a tumor-related gene specifically; and the tumor-related gene is specifically at least one of the following genes: *PPIB, p65, BIRC5, CTNNB, COPS5, CLU, EIF4E, HIF1A, TP53, VEGFA* and *SOD1.*

The advantages of the present disclosure are as follows:
1) RNAi efficiency is improved;
2) the structure is more stable, chemical stability is good, nuclease degradation resistance is enhanced, especially the stability in blood is enhanced, and a half-life period is prolonged;
3) the off-target rate is reduced;
4) a nano-particle structure may be formed, and the ability to introduce the cell is enhanced;
5) the nucleic acid molecule may be modular-designed; and
6) the polymeric nucleic acids in some structures do not require a Dicer enzyme to participate in the RNAi effect, therefore they are more resistant to chemical modification, and may be partially or completely modified.

The present disclosure is capable of enabling the CON technology to be combined with the nanotechnology, through constructing a nucleic acid structure which may be accurately designed and has self-assembly ability, thereby achieving multi-target interference, and may be used for inhibiting multiple gene expressions in a single pathway of disease occurrence or development, or simultaneously inhibiting multiple disease target gene expressions, possessing broad application prospects in multiple subject fields such as biology and chemistry.

### Brief Description of the Drawings

Fig. 1 is a schematic diagram of a targeting nucleic acid unit X-type.
Fig. 2 is a schematic diagram of a flanking nucleic acid unit H-type.
Fig. 3 is a schematic diagram of a capping-end nucleic acid unit C-type.
Fig. 4 is a plane and ring schematic diagram of an R-structure polymeric nucleic acid. The upper diagram is a plane schematic diagram of the R-structure polymeric nucleic acid. The lower diagram is a ring schematic diagram of the R-structure polymeric nucleic acid (left: R=(Xi)4; and right: R=(Xi)6).
Fig. 5 is a plane schematic diagram of an L-structure polymeric nucleic acid.
Fig. 6 is a plane schematic diagram of a Cr-structure polymeric nucleic acid.
Fig. 7 is a single-gene relative expression level.
Fig. 8 is a multi-gene relative expression level.
Fig. 9 is a multi-gene relative expression level (n=4).

### Detailed Description of the Embodiments

Unless otherwise specified, experiment methods used in the following embodiments are conventional methods.

Unless otherwise specified, materials, reagents and the like used in the following embodiments may be obtained from commercial sources.

Embodiment 1. Design and synthesis of nucleic acid units and polymeric nucleic acids

### I. Design of nucleic acid unit

The present disclosure designs three types of nucleic acid units which are respectively named as an X-type targeting nucleic acid unit, an H-type flanking nucleic acid unit, and a C-type capping-end nucleic acid unit. These nucleic acid units may be freely combined into polymeric nucleic acids in various structures.

### 1. Targeting nucleic acid unit: X-type

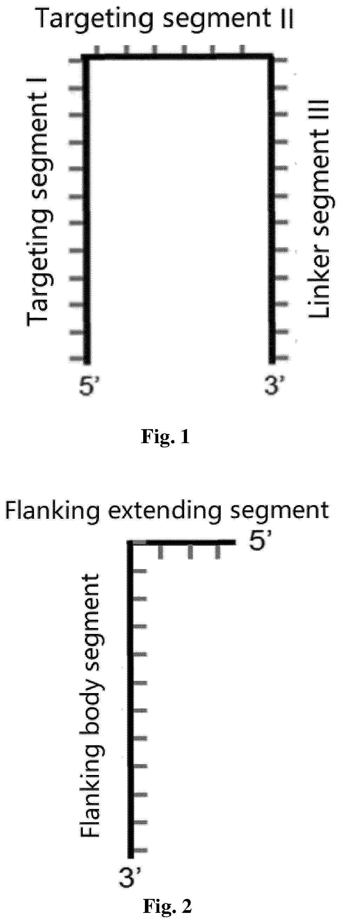

A structure of the X-type targeting nucleic acid unit is as follows: 5'-T1-T2-A3-3'. Herein T1 is a targeting segment I, T2 is a targeting segment II, A3 is a linker segment III, the T1 and T2 forms a sequence complementary to a target gene sequence. In certain specific cases, a region complementary to the target gene sequence may be extended to a partial sequence of the A3. A length of the X-type targeting nucleic acid unit is 15-50 nt, preferably 24-36 nt. Herein a length of the T1 is 5-24 nt, a length of the T2 is 1-20 nt, and a length of the A3 is 5-24 nt. The schematic diagram of the X-type targeting nucleic acid unit is as shown in Fig. 1.

### 2. Flanking nucleic acid unit: H-type

A structure of the H-type flanking nucleic acid unit is as follows: 3'-Hx-Hy-5' or 3'-Hy-Hx-5'. Herein Hx is a flanking body segment, and Hy is a flanking extending segment (the Hy may not exist). The Hx and Hy are connected by a phosphate diester bond. The Hy is positioned at 5'-end or 3'-end of the Hx. The Hx is complementary-paired with the T1 segment or the A3 segment of the X unit; and the Hy is complementary-paired with one, two or more continuous nucleotides, from the 5'-end or the 3'-end, of the T2 segment of the X unit. A length of the Hy may be 2-6 nt or longer. The schematic diagram of the H-type Flanking nucleic acid unit is as shown in Fig. 2.

### 3. Capping-end nucleic acid unit: C-type

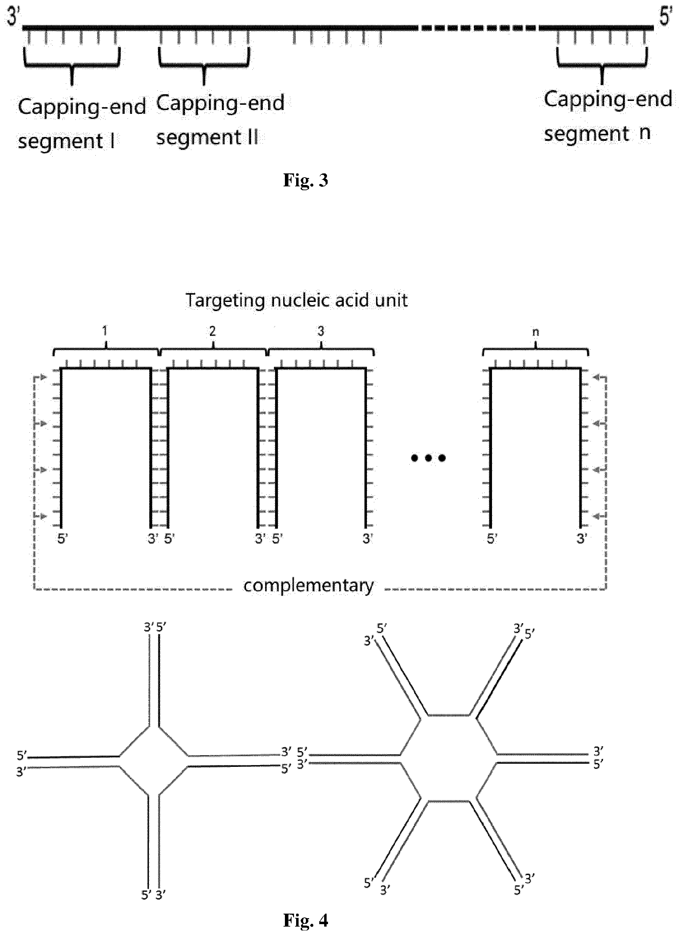

A structure of the C-type capping-end nucleic acid unit is as follows: 3'-C1-C2-......Cn-5'. Herein C1 is a capping-end segment 1, and reverse-complementary to the T2 segment of the X1 unit; C2 is a capping-end segment 2, and reverse-complementary to the T2 segment of the X2 unit, and so on, Cn is a capping-end segment n, and reverse-complementary to the T2 segment of the Xn unit. The schematic diagram of the C-type capping-end nucleic acid unit is as shown in Fig. 3.

### II. Design of polymeric nucleic acid molecules

The present disclosure designs three structures of polymeric nucleic acids according to the three nucleic acid units in the step I, which are respectively named as a R-structure polymeric nucleic acid, an L-structure polymeric nucleic acid and a Cr-structure polymeric nucleic acid. These structures may be simultaneously targeted to the different sites of the same gene, and may also be simultaneously targeted to the different sites of the different genes, to interfere with the expression of the target gene.

### 1. Polymeric nucleic acid structure I: R-structure

A structure of the R-structure polymeric nucleic acid is as follows: (Xi)n. Herein Xi is a targeting nucleic acid unit. n is an integer greater than or equal to 3, preferably the integer of 3-8, more preferably 4, 5 and 6.

The n X-type targeting nucleic acid units are respectively named as an X1 unit, an X2 unit, an X3 unit, and so on, an Xn-1 unit, and an Xn unit.

In the R-structure polymeric nucleic acid of the present disclosure, each targeting nucleic acid unit forms a double-stranded region with the other two targeting nucleic acid units in the polymeric nucleic acid except for itself through sequence complementation, namely the adjacent X units are connected by complementary pairing of the T1 segment and the A3 segment, specifically, the A3 segment of the X1 unit is complementary-paired with the T1 segment of the X2 unit, the A3 segment of the X2 unit is complementary-paired with the T1 segment of the X3 unit, and so on, the A3 segment of the Xn unit is complementary-paired with the T1 segment of the X1 unit (the T1 segment of each targeting nucleic acid unit is complementary-paired with the A3 segment of the adjacent targeting nucleic acid unit thereof, and the T1 segment of each targeting nucleic acid unit is not complementary-paired with other targeting nucleic acid unit sequences), thereby a cyclic secondary structure is formed. In the cyclic structure, each targeting nucleic acid unit has the same structure and length.

Each targeting nucleic acid unit may be combined with a specific sequence of the target gene through the T1 and T2 thereof, thereby the expression of the target gene is controlled.

The plane and ring schematic diagram of the R-structure polymeric nucleic acid is as shown in Fig. 4.

### 2. Polymeric nucleic acid structure II: L-structure

A structure of the L-structure polymeric nucleic acid is as follows: H1-(Xi)n-Hn. Herein H1 and Hn are flanking nucleic acid units, Xi is the targeting nucleic acid unit. n is an integer greater than or equal to 3, preferably the integer of 3-8, more preferably 4, 5 and 6.

The n X-type targeting nucleic acid units are respectively named as an X1 unit, an X2 unit, an X3 unit, and so on, an Xn-1 unit, and an Xn unit.

In the L-structure polymeric nucleic acid of the present disclosure, the A3 segment of the X1 unit is complementary-paired with the T1 segment of the X2 unit, the A3 segment of the X2 unit is complementary-paired with the T1 segment of the X3 unit, and so on, the A3 segment of the Xn-1 unit is complementary-paired with the T1 segment of the Xn unit; and the H1 unit is complementary-paired with the T1 segment of the X1 unit. A complementary region of the H1 unit and the X1 unit may also be extended to a part or all of the T2 segment of the X1 unit; the Hn unit is complementary-paired with the A3 segment of the Xn unit, and a complementary region of the Hn unit and the Xn unit may also be extended to a part or all of the T2 segment of the Xn unit. The 5'-end of the H1 unit or the 3'-end of the Hn unit further includes a Hy segment, the Hy segment is complementary-paired with one, two or more continuous nucleic acid molecules, from the 5'-end or the 3'-end, of the targeting segment II of the X1 unit or the Xn unit.

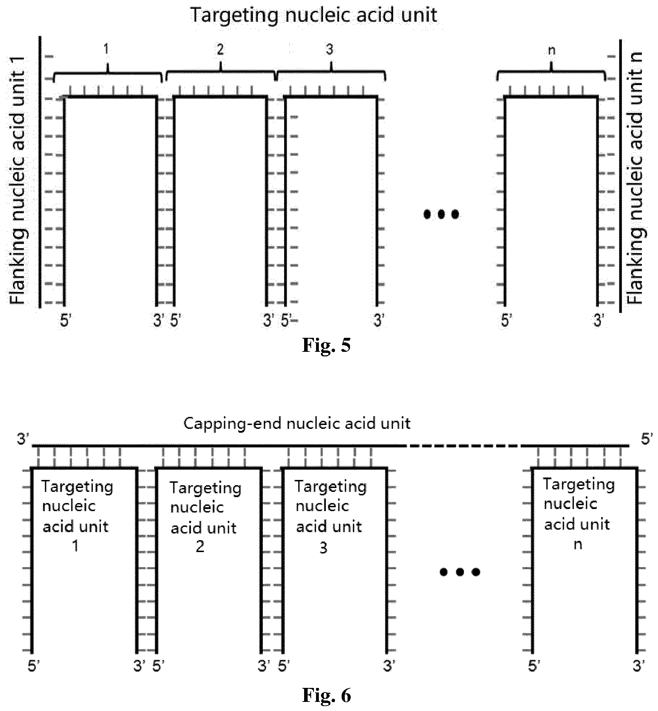

The plane schematic diagram of the L-structure polymeric nucleic acid is as shown in Fig. 5.

### 3. Polymeric nucleic acid structure III: Cr-structure

A structure of the Cr-structure polymeric nucleic acid is as follows: (Xi)n-C. Herein Xi is a targeting nucleic acid unit, and C is a capping-end nucleic acid unit. n is an integer greater than or equal to 3, preferably the integer of 3-8, more preferably 4, 5 and 6.

The n X-type targeting nucleic acid units are respectively named as an X1 unit, an X2 unit, an X3 unit, and so on, an Xn-1 unit, and an Xn unit.

In the Cr-structure polymeric nucleic acid of the present disclosure, the A3 segment of the X1 unit is complementary-paired with the T1 segment of the X2 unit, the A3 segment of the X2 unit is complementary-paired with the T1 segment of the X3 unit, and so on, the A3 segment of the Xn-1 unit is complementary-paired with the T1 segment of the Xn unit; the capping-end nucleic acid unit C is formed by a capping-end segment 1, a capping-end segment 2, and so on, a capping-end segment n, the capping-end segment 1 is complementary-paired with the T2 segment of the X1 unit, the capping-end segment 2 is complementary-paired with the T2 segment of the X2 unit, and so on, the capping-end segment n is complementary-paired with the T2 segment of the Xn unit, the capping-end segment 1, the capping-end segment 2, and so on, and the capping-end segment n are connected into a single-stranded nucleic acid through a phosphate diester bond.

The plane schematic diagram of the Cr-structure polymeric nucleic acid is as shown in Fig. 6.

### III. Synthetic method of polymeric nucleic acid and modification method of nucleic acid unit

### 1. Synthetic method of polymeric nucleic acid

The nucleic acid units of the present disclosure are annealed each other in a sequence specificity mode, the complementarity thereof promotes self-assembly of this polymeric nucleic acid, and a polymeric nucleic acid molecule with a secondary structure is formed. The specific synthetic method is as follows:
1) the nucleic acid units required by a polymeric nucleic acid structure is synthesized; and
2) the nucleic acid units are placed in an annealing condition, and annealed mutually, to form a secondary structure body by the self-assembly.

A reaction system for annealing is a system obtained by uniformly mixing each nucleic acid unit in equal molar weight, RNA annealing buffer (5X) (Biyuntian Annealing Buffer for RNA oligos (5X), R0051) and water (DEPC water).

A reaction condition for annealing is that a temperature is 90 DEG C in a polymerase chain reaction (PCR) meter, it is adequately denaturized in 2 min, and then the temperature in the PCR meter is reduced to 25 DEG C so that it is annealed.

### 2. Modification method of the nucleic acid units

In order to increase stability and biological activity of a nuclease, suitable modifications of nucleotide sugar, base and phosphate moieties may be introduced into the nucleic acid, including chemical modification of ribose, such as a 2' ribose hydroxyl group is substituted by a halogen group or an O-alkyl group, the alkyl group includes a methyl, an ethyl, a propyl or a methylethyl and the like, such nucleic acid modification may not reduce the interference activity of the polymeric nucleic acid. The nucleic acid units designed by the present disclosure include the following modifications.
1) The ribose 2-site hydroxyl groups of 5-9 continuous nucleotides, from the first nucleotide at the 3'-end, of the linker segment A3 in the targeting nucleic acid unit are substituted by the halogen group or the O-alkyl group.
2) The ribose 2-site hydroxyl groups of 5-9 continuous nucleotides, from the first nucleotide at the 3'-end, of the flanking body segment of the flanking nucleic acid unit are substituted by the halogen group or the O-alkyl group; or the ribose 2-site hydroxyl groups of 8-30 continuous nucleotides, from the first nucleotide at the 3'-end, of the flanking nucleic acid unit are substituted by the halogen group or the O-alkyl group. Preferably, the ribose 2-site hydroxyl groups of 14-18 continuous nucleotides, from the first nucleotide at the 3'-end, of the flanking nucleic acid unit are substituted by the halogen group or the O-alkyl group.
3) The ribose 2-site hydroxyl groups of 2-6 continuous nucleotides, from the 5'-end to the 3'-end, of each capping-end segment of the capping-end nucleic acid unit are substituted by the halogen group or the O-alkyl group.

Embodiment 2. Preparation of polymeric nucleic acid and application thereof in interference of target gene expression

### (I) R-structure polymeric nucleic acid: inhibition experiment targeting to different regions of a same gene

### 1. Polymeric nucleic acid

A polymeric nucleic acid in the following structure is prepared, a targeting nucleic acid unit of each structure is respectively targeting to 4, 5 or 6 different regions of *VEGFA* and *TP53* genes.

A represents that T1 and A3 segments of the targeting nucleic acid unit are 12 nt, and a T2 segment is 6 nt.
B represents that T1 and A3 segments of the targeting nucleic acid unit are 12 nt, and a T2 segment is 5 nt.
C represents that T1 and A3 segments of the targeting nucleic acid unit are 12 nt, and a T2 segment is 3 nt.
D represents that T1 and A3 segments of the targeting nucleic acid unit are 11 nt, and a T2 segment is 6 nt.
E represents that T1 and A3 segments of the targeting nucleic acid unit are 10 nt, and a T2 segment is 6 nt.

While n is 4, the annealing system is as follows (a total volume is 100µL): 20µL of each nucleic acid unit solution (the concentration is 100µM), 15µL of the RNA annealing buffer (5X), and 5µL of the DEPC water.

While n is 5, the annealing system is as follows (the total volume is 150µL): 20µL of each nucleic acid unit solution (the concentration is 150µM), 30µL of the RNA annealing buffer (5X), and 20µL of the DEPC water.

While n is 6, the annealing system is as follows (the total volume is 200µL): 20µL of each nucleic acid unit solution (the concentration is 200µM), 40µL of the RNA annealing buffer (5X), and 40µL of the DEPC water.

The polymeric nucleic acid of which the target gene is *VEGFA* is the following 1)-11):
1) VEGFA-R6-A (R-structure, n=6): an X unit sequence thereof is sequences 42-47 in Table 1 successively from X1-X6.
2) VEGFA-R6-B (R-structure, n=6): an X unit sequence thereof is sequences 48-53 in Table 1 successively from X1-X6.
3) VEGFA-R6-C (R-structure, n=6): an X unit sequence thereof is sequences 54-59 in Table 1 successively from X1-X6.
4) VEGFA-R5-A (R-structure, n=5): an X unit sequence thereof is sequences 42, 43, 45, 46 and 47 in Table 1 successively from X1-X5.
5) VEGFA-R5-B (R-structure, n=5): an X unit sequence thereof is sequences 48, 49, 51, 52 and 53 in Table 1 successively from X1-X5.
6) VEGFA-R5-C (R-structure, n=5): an X unit sequence thereof is sequences 54, 55, 57, 58 and 59 in Table 1 successively from X1-X5.
7) VEGFA-R4-A (R-structure, n=4): an X unit sequence thereof is sequences 42, 45, 46 and 47 in Table 1 successively from X1-X4.
8) VEGFA-R4-B (R-structure, n=4): an X unit sequence thereof is sequences 48, 51, 52 and 53 in Table 1 successively from X1-X4.
9) VEGFA-R4-C (R-structure, n=5): an X unit sequence thereof is sequences 54, 57, 58 and 59 in Table 1 successively from X1-X4.
10) VEGFA-R6-D (R-structure, n=6): an X unit sequence thereof is sequences 60-65 in Table 1 successively from X1-X6.
11) VEGFA-R6-E (R-structure, n=6): an X unit sequence thereof is sequences 66-71 in Table 1 successively from X1-X6.

The polymeric nucleic acid of which the target gene is *TP53* is the following 1)-11):
1) TP53-R6-A (R-structure, n=4): an X unit sequence thereof is sequences 72-77 in Table 2 successively from X1-X6.
2) TP53-R6-B (R-structure, n=6): an X unit sequence thereof is sequences 78-83 in Table 2 successively from X1-X6.
3) TP53-R6-C (R-structure, n=6): an X unit sequence thereof is sequences 84-89 in Table 2 successively from X1-X6.
4) TP53-R5-A (R-structure, n=5): an X unit sequence thereof is sequences 73-77 in Table 2 successively from X1-X5.
5) TP53-R5-B (R-structure, n=5): an X unit sequence thereof is sequences 79-83 in Table 2 successively from X1-X5.
6) TP53-R5-C (R-structure, n=5): an X unit sequence thereof is sequences 85-89 in Table 2 successively from X1-X5.
7) TP53-R4-A (R-structure, n=4): an X unit sequence thereof is sequences 74-77 in Table 2 successively from X1-X4.
8) TP53-R4-B (R-structure, n=4): an X unit sequence thereof is sequences 80-83 in Table 2 successively from X1-X4.
9) TP53-R4-C (R-structure, n=5): an X unit sequence thereof is sequences 86-89 in Table 2 successively from X1-X4.
10) TP53-R6-D (R-structure, n=6): an X unit sequence thereof is sequences 90-95 in Table 2 successively from X1-X6.
11) TP53-R6-E (R-structure, n=6): an X unit sequence thereof is sequences 96-101 in Table 2 successively from X1-X6.

**Table 1. Sequences of nucleic acid units**

| Target gene | Sequence (5'-3') | SEQ ID NO: |
|---|---|---|
| *VEGFA* | AGCAGAAAGUUCAUGGUUUCUUGggugcau | 42 |
| *VEGFA* | AUGCACCCAAGACAGCAGAGAUCgaguaca | 43 |
| *VEGFA* | UGUACUCGAUCUCAUCAGGGUGGacaucuu | 44 |
| *VEGFA* | AAGAUGUCCACCAGGGUCAUGCGgaucaaa | 45 |
| *VEGFA* | UUUGAUCCGCAUAAUCUGGGCCAgcacaua | 46 |
| *VEGFA* | UAUGUGCUGGCCUUGGUGGAACUuucugcu | 47 |
| *VEGFA* | AGCAGAAAGUUCAUGGUUCUUGggugcau | 48 |
| *VEGFA* | AUGCACCCAAGACAGCAAGAUCgaguaca | 49 |
| *VEGFA* | UGUACUCGAUCUCAUCAGGUGGacaucuu | 50 |
| *VEGFA* | AAGAUGUCCACCAGGGUAUGCGgaucaaa | 51 |
| *VEGFA* | UUUGAUCCGCAUAAUCUGGCCAgcacaua | 52 |
| *VEGFA* | UAUGUGCUGGCCUUGGUGAACUuucugcu | 53 |
| *VEGFA* | AGCAGAAAGUUCAUGUCUUGggugcau | 54 |
| *VEGFA* | AUGCACCCAAGACAGAGAUCgaguaca | 55 |
| *VEGFA* | UGUACUCGAUCUCAUGGUGGacaucuu | 56 |
| *VEGFA* | AAGAUGUCCACCAGGAUGCGgaucaaa | 57 |
| *VEGFA* | UUUGAUCCGCAUAAUGGCCAgcacaua | 58 |
| *VEGFA* | UAUGUGCUGGCCUUGGAACUuucugcu | 59 |
| *VEGFA* | AGCAGAAAGUUCAUGGUCUUGggugcau | 60 |
| *VEGFA* | AUGCACCCAAGACAGCAGAUCgaguaca | 61 |
| *VEGFA* | UGUACUCGAUCUCAUCAGUGGacaucuu | 62 |
| *VEGFA* | AAGAUGUCCACCAGGGUUGCGgaucaaa | 63 |
| *VEGFA* | UUUGAUCCGCAUAAUCUGCCAgcacaua | 64 |
| *VEGFA* | UAUGUGCUGGCCUUGGUAACUuucugcu | 65 |
| *VEGFA* | AGCAGAAAGUUCAUGGUUGggugcau | 66 |
| *VEGFA* | AUGCACCCAAGACAGCAUCgaguaca | 67 |
| *VEGFA* | UGUACUCGAUCUCAUCUGGacaucuu | 68 |
| *VEGFA* | AAGAUGUCCACCAGGGGCGgaucaaa | 69 |
| *VEGFA* | UUUGAUCCGCAUAAUCCCAgcacaua | 70 |
| *VEGFA* | UAUGUGCUGGCCUUGGACUuucugcu | 71 |

**Table 2. Nucleic acid unit sequences**

| Target gene | Sequence (5'-3') | SEQ ID NO: |
|---|---|---|
| *TP53* | UGUGGAAUCAACCCACAGUUUGCgugugga | 72 |
| *TP53* | UCCACACGCAAAUUUCCUACAGAaacacuu | 73 |
| *TP53* | AAGUGUUUCUGUCAUCCAACUACaugugua | 74 |
| *TP53* | UACACAUGUAGUUGUAGUUGGUAaucuacu | 75 |
| *TP53* | AGUAGAUUACCACUGGAGUCUCCgcaagaa | 76 |
| *TP53* | UUCUUGCGGAGAUUCUCUGUUGAuuccaca | 77 |
| *TP53* | UGUGGAAUCAACCCACAUUUGCgugugga | 78 |
| *TP53* | UCCACACGCAAAUUUCCACAGAaacacuu | 79 |
| *TP53* | AAGUGUUUCUGUCAUCCACUACaugugua | 80 |
| *TP53* | UACACAUGUAGUUGUAGUGGUAaucuacu | 81 |
| *TP53* | AGUAGAUUACCACUGGAUCUCCgcaagaa | 82 |
| *TP53* | UUCUUGCGGAGAUUCUCGUUGAuuccaca | 83 |
| *TP53* | UGUGGAAUCAACCCAUUUGCgugugga | 84 |
| *TP53* | UCCACACGCAAAUUUACAGAaacacuu | 85 |
| *TP53* | AAGUGUUUCUGUCAUACUACaugugua | 86 |
| *TP53* | UACACAUGUAGUUGUUGGUAaucuacu | 87 |
| *TP53* | AGUAGAUUACCACUGUCUCCgcaagaa | 88 |
| *TP53* | UUCUUGCGGAGAUUCGUUGAuuccaca | 89 |
| *TP53* | UGUGGAAUCAACCCACAUUGCgugugga | 90 |
| *TP53* | UCCACACGCAAAUUUCCCAGAaacacuu | 91 |
| *TP53* | AAGUGUUUCUGUCAUCCCUACaugugua | 92 |
| *TP53* | UACACAUGUAGUUGUAGGGUAaucuacu | 93 |
| *TP53* | AGUAGAUUACCACUGGACUCCgcaagaa | 94 |
| *TP53* | UUCUUGCGGAGAUUCUCUUGAuuccaca | 95 |
| *TP53* | UGUGGAAUCAACCCACUGCgugugga | 96 |
| *TP53* | UCCACACGCAAAUUUCAGAaacacuu | 97 |
| *TP53* | AAGUGUUUCUGUCAUCUACaugugua | 98 |
| *TP53* | UACACAUGUAGUUGUAGUAaucuacu | 99 |
| *TP53* | AGUAGAUUACCACUGGUCCgcaagaa | 100 |
| *TP53* | UCUUGCGGAGAUUCUUGAuuccaca | 101 |

| | | |
|---|---|---|
| Note: lower case letters represent that the nucleotide ribose is modified by 2'-O-methyl ribose. The underlined sequence is a target sequence. | | |

### 2. Inhibition experiment

5×10⁵ HeLa cells (an ATCC number is CRL-1958) are inoculated in a 12-pore culture plate of a DMEM culture medium containing 10% of fetal bovine serum, and the polymeric nucleic acid prepared in the step 1 is used to respectively transfect the HeLa cells: after the polymeric nucleic acid is mixed with a transfection reagent and buffer (GUANGZHOU RIBOBIO CO., LTD., named as riboFECT™ CP Buffer, and Article No. C10511-1), it is added to the cell culture medium, a volume of each pore is 1 mL, so that the final transfection concentration of the polymeric nucleic acid is 50 nM, and the culture plate is placed in an incubator with 5% of CO₂ and 37 DEG C of a constant temperature, and cultured for 48 h. The transfection reagent and specific steps of transfection refer to a method in riboFect™ (GUANGZHOU RIBOBIO CO., LTD).

In addition to a test group, a negative control group (NC) and an untreated control group (NT) are also set for each time of cell plating. There are 3 replicates in the test group and the control groups. A control sequence of the NC group is siRNA, and is a double-stranded RNA molecule obtained by complementary-binding of the following two single-stranded RNA molecules: 5'-uucuccgaacgugucacgudTdT-3' and 5'-acgugacacguucggagaadtdt -3'.

In 37 DEG C and 5% of the CO₂, after being incubated for 48 h, transfected cells are collected, RNAs of the transfected cells are extracted by a Trizol method and a real-time quantitative PCR is performed, an mRNA expression level of the target gene is detected, q-PCR is repeated 3 times, and all of results are represented by an average value±SD. Sequences of real-time quantitative PCR primers for detecting the target genes are as shown in the attached table.

Detection results are as shown in Table 3. It is indicated from an experiment result that the above polymer molecules in different structures may efficiently inhibit the expression of the target gene.

**Table 3. mRNA relative expression level**

| | *VEGFA* | *TP53* |
|---|---|---|
| R6-A | 0.05 | 0.I0 |
| R6-B | 0.02 | 0.06 |
| R6-C | 0.13 | 0.21 |
| R5-A | 0.06 | 0.06 |
| R5-B | 0.03 | 0.02 |
| R5-C | 0.12 | 0.12 |
| R4-A | 0.04 | 0.04 |
| R4-B | 0.06 | 0.01 |
| R4-C | 0.23 | 0.13 |
| R6-D | 0.06 | 0.22 |
| R6-E | 0.06 | 0.07 |

| | | |
|---|---|---|
| Note: the negative control expression level is 1. | | |

### (II) R-structure polymeric nucleic acid: low-concentration inhibition experiment 1. Polymeric nucleic acid

A step of preparing an X unit sequence of the polymeric nucleic acid VEGFA-R6-A and an X unit sequence of the polymeric nucleic acid TP53-R6-A is the same as the step (I); and an X unit sequence of the polymeric nucleic acid P65-R6-A prepared is successively sequences 15, 16, 17, 23, 24 and 25 in Table 5 from X1-X6.

### 2. Inhibition experiment

Only the final transfection concentration of the polymeric nucleic acid in the inhibition experiment in the step (I) is changed to 1 nM, and the other steps are not changed.

A detection result is as shown in Table 4. It is indicated from an experiment result that the polymeric nucleic acid of the present disclosure with the low-concentration may also achieve a purpose of reducing the expression level of the target gene.

**Table 4. mRNA relative expression level**

| | *VEGFA* | *TP53* | *P65* |
|---|---|---|---|
| R6-A (1nM) | 0.53 | 0.64 | 0.51 |

| | | | |
|---|---|---|---|
| Note: the negative control expression level is 1. | | | |

### (III) Three structures of polymeric nucleic acids: inhibition experiment targeted to different regions of a same gene

### 1. Polymeric nucleic acids

The polymeric nucleic acids in the following structures are prepared, a targeting nucleic acid unit of each structure is respectively targeted to 4 or 6 different regions of *PPIB* or *P65* gene.

The polymeric nucleic acid of which the target gene is *PPIB* is the following 1)-5):
1) PPIB-R4 (R-structure, n=4): an X unit sequence thereof is successively sequences 1-4 in Table 5 from X1-X4.
2) PPIB-L4 (L-structure, n=4): an X unit sequence thereof is successively sequences 1-3 and the sequence 5 in Table 5 from X1-X4, an H1 unit sequence thereof is the sequence 20, and an H4 unit sequence thereof is the sequence 21.
3) PPIB-Cr4 (Cr-structure, n=4): an X unit sequence thereof is successively sequences 1-4 in Table 5 from X1-X4, and a C unit sequence thereof is the sequence 8.
4) PPIB-R6 (R-structure, n=6): an X unit sequence thereof is successively sequences 2, 3, 9, 10, 11 and 12 in Table 5 from X1-X6.
5) PPIB-L6 (L-structure, n=6): an X unit sequence thereof is successively sequences 2, 3, 9, 10, 11 and 13 in Table 5 from X1-X6, an H1 unit sequence thereof is the sequence 6, and an H6 unit sequence thereof is the sequence 14.

The polymeric nucleic acid of which the target gene is *P65* is the following 1)-5):
1) P65-R4 (R-structure, n=4): an X unit sequence thereof is successively sequences 15-18 in Table 5 from X1-X4.
2) P65-L4 (L-structure, n=4): an X unit sequence thereof is successively sequences 15-17 and the sequence 19 in Table 5 from X1-X4, an H1 unit sequence thereof is the sequence 6, and an H4 unit sequence thereof is the sequence 7.
3) P65-Cr4 (Cr-structure, n=4): an X unit sequence thereof is successively sequences 15-18 in Table 5 from X1-X4, and a C unit sequence thereof is the sequence 22.
4) P65-R6 (R-structure, n=6): an X unit sequence thereof is successively sequences 15, 16, 17, 23, 24 and 25 in Table 5 from X1-X6.
5) P65-L6 (L-structure, n=6): an X unit sequence thereof is successively sequences 15, 16, 17, 23, 24 and 26 in Table 5 from X1-X6, an H1 unit sequence thereof is the sequence 20, and an H6 unit sequence thereof is the sequence 27.

**Table 5. Sequences of nucleic acid units**

| Target gene | Sequence (5'-3') | SEQ ID NO: |
|---|---|---|
| *PPIB* | AGAUGCUCUUUCCUCCUGCAAGGuguauuu | 1 |
| *PPIB* | AAAUACACCUUGACGGUGGAUGAagaugua | 2 |
| *PPIB* | UACAUCUUCAUCUCCAAUUCUCUucggaaa | 3 |
| *PPIB* | UUUCCGAAGAGACCAAAGGAAAGagcaucu | 4 |
| *PPIB* | UUUCCGAAGAGACCAAAGUCUACgagaaag | 5 |
| *PPIB* | GGAGGAAAGagcaucu | 6 |
| *PPIB* | cuuucucguagacuuu | 7 |
| *PPIB* | cuuuGGauugGAcaccGUcaggAG | 8 |
| *PPIB* | AGAUGCUCUUUCCUCCUGCAUGAaggugcu | 9 |
| *PPIB* | AGCACCUUCAUGUUGCGUCAAGGuguauuu | 10 |
| *PPIB* | UUUCCGAAGAGACCAAAGCGUGUaaucaag | 11 |
| *PPIB* | CUUGAUUACACGAUGGAAGAAAGagcaucu | 12 |
| *PPIB* | CUUGAUUACACGAUGGAAUCUACgagaaag | 13 |
| *PPIB* | cuuucucguagauucc | 14 |
| *P65* | UGUGUAGCCAUUGAUCUUGCAUCaugaaga | 15 |
| *P65* | UCUUCAUGAUGCUCUUGAAUACCaccaaga | 16 |
| *P65* | UCUUGGUGGUAUCUGUGCUCGUCaccggau | 17 |
| *P65* | AUCCGGUGACGAUCGUCUAAUGGcuacaca | 18 |
| *P65* | AUCCGGUGACGAUCGUCUACACAucgguaa | 19 |
| *P65* | GAUCAAUGGcuacaca | 20 |
| *P65* | uuaccgauguguagac | 21 |
| *P65* | agacGAgcacAGucaaGAaagaUC | 22 |
| *P65* | AUCCGGUGACGAUCGUCUUCAGGagaugaa | 23 |
| *P65* | UUCAUCUCCUGAAAGGAGAUCAGcuccuaa | 24 |
| *P65* | UUAGGAGCUGAUCUGACUAAUGGcuacaca | 25 |
| *P65* | UUAGGAGCUGAUCUGACUACACAucgguaa | 26 |
| *P65* | uuaccgauguguaguc | 27 |

| | | |
|---|---|---|
| Note: lower case letters represent that the nucleotide ribose is modified by 2-O-methyl ribose. The underlined sequence is a target sequence. X unit: the 1-12th sites are T1; the 13-18th sites are T2; and the 19-30th sites are A3. H1 unit: the 1-4th sites are Hy; and the 5-16th sites are Hx. H4 unit: the 1-12th sites are Hx; and the 13-16th sites are Hy. C unit: the 1-6th sites are C4, the 7-12th sites are C3, the 13-18th sites are C2, and the 19-24th sites are C1. | | |

### 2. Inhibition experiment

Steps of the inhibition experiment are the same as the step (I).

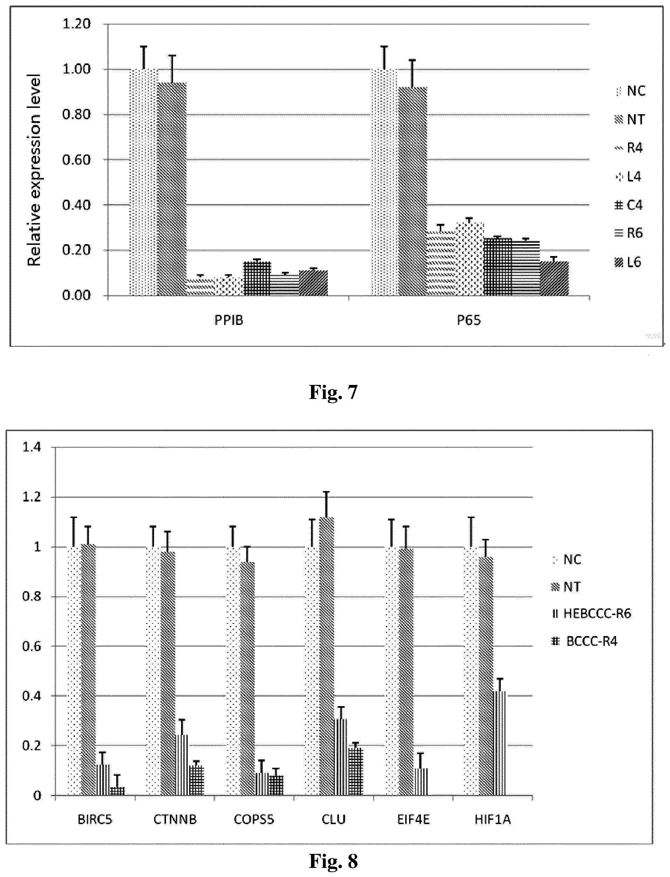

An inhibition result is as shown in Fig. 7. It is indicated from the result that 5 structures of the polymeric nucleic acids effectively inhibit the expression of the target genes. Inhibition levels in allusion to the target gene *PPIB* are above 80%, and the inhibition levels in allusion to the target gene *P65* are above 75%.

### (IV) Inhibition experiment of R-structure polymeric nucleic acid targeted to different genes (n=4 and 6)

### 1. Polymeric nucleic acid

A polymeric nucleic acid in the following R-structure is prepared, and the polymeric nucleic acid is targeted to 4 or 6 different target genes.

BCCC-R4: the target genes are *BIRC5, CTNNB, COPS5* and *CLU*, an X unit sequence thereof is successively sequences 28-31 in Table 3 from X1-X4.

BCCCEH-R6: the target genes are *BIRC5, CTNNB, COPS5, CLU, EIF4E* and *HIF1A,* an X unit sequence thereof is successively sequences 32, 33, 28, 29, 30 and 34 in Table 6 from X1-X6.

### 2. Inhibition experiment

Steps of the inhibition experiment are the same as the step (I).

**Table 6. Sequences of nucleic acid units**

| Target gene | Sequence (5'-3') | SEQ ID NO: |
|---|---|---|
| *BIRC5* | AGAAGAAACACUGGGCCAACUGCugaucuu | 28 |
| *CTNNB1* | AAGAUCAGCAGUCUCAUUUGGUCaggucuu | 29 |
| *COPS5* | AAGACCUGACCAGUGGUAUUUGAcucugau | 30 |
| *CLU* | AUCAGAGUCAAAGAGCUUAGUGUuucuucu | 31 |
| *HIF1A* | UCAAGUUGCUGGUCAUCAGGAGGuugcuaa | 32 |
| *EIF4E* | UUAGCAACCUCCUGAUUAAGUGUuucuucu | 33 |
| *CLU* | AUCAGAGUCAAAGAGCUUCCAGCaacuuga | 34 |

| | | |
|---|---|---|
| Note: lower case letters represent that the nucleotide ribose is modified by 2-O-methyl ribose. The underlined sequence is a target sequence. X unit: the 1-12th sites are T1; the 13-18th sites are T2; and the 19-30th sites are A3. | | |

A result of the inhibition experiment is as shown in Fig. 8. It is indicated from the result that 2 structures of the polymeric nucleic acids effectively inhibit the expressions of 4 or 6 target genes. Expression levels of the polymeric nucleic acid in R4-structure targeted to 4 genes are reduced below 0.2.

### (V) Inhibition experiment of polymeric nucleic acids in R-structure, L-structure and Cr-structure targeted to different genes (n=4)

### 1. Polymeric nucleic acid

A polymeric nucleic acid in each following structure is prepared, and the polymeric nucleic acid is targeted to 4 different target genes.

SPPV-R4: the target genes are *SOD1, PPIB, P65,* and *VEGFA,* an X unit sequence thereof is successively sequences 35-38 in Table 7 from X1-X4.

SPPV-L4: the target genes are *SOD1, PPIB, P65,* and *VEGFA,* an X unit sequence thereof is successively sequences 35-38 in Table 7 from X1-X4, an H1 unit sequence thereof is the sequence 39, and an H4 unit sequence thereof is the sequence 40.

SPPV-Cr4: the target genes are *SOD1, PPIB, P65,* and *VEGFA,* an X unit sequence thereof is successively sequences 35-38 in Table 7 from X1-X4, and a C unit sequence thereof is the sequence 41.

### 2. Inhibition experiment

It is the same as the inhibition experiment in the step (I).

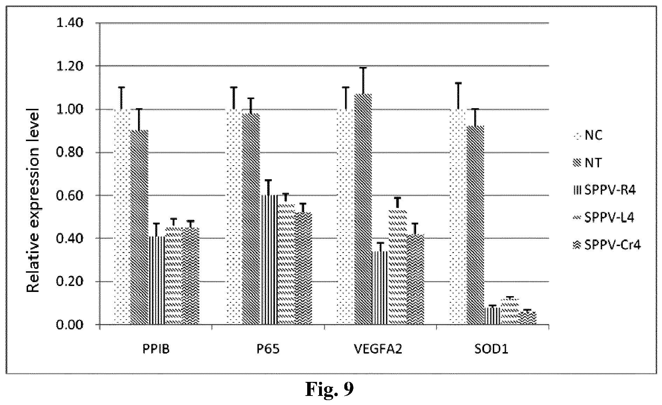

A result of the inhibition experiment is as shown in Fig. 9. It is indicated from the result that 3 structures of the polymeric nucleic acids effectively inhibit the expressions of 4 target genes. It is also discovered that in three polymeric structures, the inhibition effect of the polymeric nucleic acid in Cr4-structure is better, and an expression level of each target gene thereof is the lowest.

**Table 7. Sequences of nucleic acid units**

| Target gene | Sequence (5'-3') | SEQ ID NO: |
|---|---|---|
| *SOD1* | UACUUUCUUCAUUUCCACCUGUUccaaaaa | 35 |
| *PPIB* | UUUUUGGAACAGUCUUUCUGAGAccuucaa | 36 |
| *P65* | UUGAAGGUCUCAUAUGUCCAUGCagauuau | 37 |
| *VEGFA* | AUAAUCUGCAUGGUGAUGAUGAAgaaagua | 38 |
| H | GGAAAUGAAgaaagua | 39 |
| H4 | uacuuucuucaucauc | 40 |
| C | caucACgacaUAgaaaGAguggAA | 41 |

| | | |
|---|---|---|
| Note: lower case letters represent that the nucleotide ribose is modified by 2-O-methyl ribose. The underlined sequence is a target sequence. X unit: the 1-12th sites are T1; the 13-18th sites are T2; and the 19-30th sites are A3. H1 unit: the 1-4th sites are Hy; and the 5-16th sites are Hx. H4 unit: the 1-12th sites are Hx; and the 13-16th sites are Hy. C unit: the 1-6th sites are C4, the 7-12th sites are C3, the 13-18th sites are C2, and the 19-24th sites are C1. | | |

Sequences of real-time quantitative PCR primers for detecting the target genes in each of the above experiments are as shown in Table 8.

**Table 8. Sequences of real-time quantitative PCR primers for detecting target genes**

| Primer name | Primer sequence (5'-3') |
|---|---|
| H-TP53-qPCR-F | ttgtgcctgtcctgggagag |
| H-TP53-qPCR-R | ggagaggagctggtgttgttg |
| H-HIF1 A-qPCR-F | gccctaacgtgttatctgtc |
| H-HIF1 A-qPCR-R | cgctttctctgagcattctg |
| h-EIF4E-qPCR-F | gg ag gttg ctaacccag aacac |
| h-EIF4E-qPCR-R | ggagatcagccgcaggtttg |
| h-VEGFA-qPCR-F | gagggcagaatcatcacgaag |
| h-VEGFA-qPCR-R | actcg atctcatcag ggtactc |
| h-PPIB-qPCR-F | ggcaagcatgtggtgtttgg |
| h-PPIB-qPCR-R | ggtttatcccggctgtctgtc |
| h-p65-qPCR-F | gggaaggaacgctgtcagag |
| h-p65-qPCR-R | tagcctcagggtactccatca |
| h-SOD1-qPCR-F | gcagggcatcatcaatttcg |
| h-SOD1-qPCR-R | gaatccatgcaggccttcag |
| H-B I RC5-q PCR-F | agaactggcccttcttggag |
| H-BIRC5-qPCR-R | gaaacactgggccaagtctg |
| H-CTNNB1-qPCR-F | gctcgggatgttcacaacc |
| H-CTNNB1-qPCR-R | ccctgcagctactctttgg |
| H-COPS5-qPCR-F | tggaataaatactgggtgaatacg |
| H-COPS5-qPCR-R | ggcttctgactgctctaac |
| H-CLU-qPCR-F | caaggcgaagaccagtactatc |
| H-CLU-qPCR-R | cagtgacaccggaaggaac |

### Industrial Application

The present disclosure provides a new-type nucleic acid unit for constructing a polymeric nucleic acid and the polymeric nucleic acid for interfering with an expression of a target gene. The present disclosure is capable of, through designing and constructing the new-type nucleic acid unit and the self-assembled polymeric nucleic acid thereof, achieving multi-target interference, and may be used for inhibiting multiple gene expressions in a signaling pathway of disease occurrence or development, or simultaneously inhibiting multiple disease target gene expressions, possessing broad application prospects in multiple subject fields such as biology and chemistry. The polymeric nucleic acid may target multiple sequences simultaneously, herein the sequences may be located in one gene, or located in multiple genes. The advantages of the present disclosure include: 1) a high RNAi performance; 2) good stability; 3) reduction of an off-target rate; 4) enhanced ability to be introduced into cells; and 5) a modular design.

## Claims

1. A polymeric nucleic acid molecule for interfering with an expression of a target gene, wherein the polymeric nucleic acid molecule is formed by n X-type nucleic acid molecules;
each of the X-type nucleic acid molecules is formed by a targeting segment I, a targeting segment II and a linker segment III successively from a 5'-end;
the targeting segment I of each of the X-type nucleic acid molecules is complementary-paired with the linker segment III of an adjacent X-type nucleic acid molecule thereof;
the targeting segment I and the targeting segment II of each of the X-type nucleic acid molecules is complementary-paired with a target gene;
a length of each of the X-type nucleic acid molecules is the same; and
the n is an integer greater than or equal to 3.

2. The polymeric nucleic acid molecule as claimed in claim 1, wherein the polymeric nucleic acid molecule further comprises a H-type nucleic acid molecule; the H-type nucleic acid molecule is formed by a H1-type nucleic acid molecule and a Hn-type nucleic acid molecule;
the n X-type nucleic acid molecules are respectively named as an X1 unit, an X2 unit, an X3 unit, and so on, an Xn-1 unit, and an Xn unit;
the linker segment III of the X1 unit is complementary-paired with the targeting segment I of the X2 unit, the linker segment III of the X2 unit is complementary-paired with the targeting segment I of the X3 unit, and so on, the linker segment III of the Xn-1 unit is complementary-paired with the targeting segment I of the Xn unit;
the H1-type nucleic acid molecule is complementary-paired with the targeting segment I of the X1 unit, and the Hn-type nucleic acid molecule is complementary-paired with the linker segment III of the Xn unit.

3. The polymeric nucleic acid molecule as claimed in claim 2, wherein
5'-end or 3'-end of the H-type nucleic acid molecule further comprises a Hy segment; and the H-type nucleic acid molecule is formed by an Hx segment and the Hy segment;
the Hx segment of the H1-type nucleic acid molecule is complementary-paired with the targeting segment I of the X1 unit; the Hx segment of the Hn-type nucleic acid molecule is complementary-paired with the linker segment III of the Xn unit; and
the Hy segment is complementary-paired with one, two or more continuous nucleic acid molecules of the targeting segment II of the X1 unit or the Xn unit from 5'-end or 3'-end.

4. The polymeric nucleic acid molecule as claimed in claim 1, wherein the polymeric nucleic acid molecule further comprises a C-type nucleic acid molecule;
the C-type nucleic acid molecule is formed by n segments connected successively, the n segments are respectively reverse complementary sequences of the targeting segment II in the n X-type nucleic acid molecules.

5. The polymeric nucleic acid molecule as claimed in any one of claims 1-4, wherein the X-type nucleic acid molecule, the H-type nucleic acid molecule and the C-type nucleic acid molecule are a single-stranded RNA molecule.

6. The polymeric nucleic acid molecule as claimed in claim 1 or 3, wherein
a length of the X-type nucleic acid molecule is 15-50 nt;
a length of the targeting segment I is 5-24 nt;
a length of the targeting segment II is 1-20 nt;
a length of the linker segment III is 5-24 nt; and
a sum of the lengths of the targeting segment I and the targeting segment II is 14-16 nt at least.

7. The polymeric nucleic acid molecule as claimed in claim 6, wherein the length of the X-type nucleic acid molecule is 24-36 nt.

8. The polymeric nucleic acid molecule as claimed in claim 1, wherein the polymeric nucleic acid molecule at least comprises a modified nucleotide.

9. The polymeric nucleic acid molecule as claimed in claim 8, wherein a modification is a phosphoric acid backbone modification, a base modification and/or a ribose modification.

10. The polymeric nucleic acid molecule as claimed in claim 9, wherein the ribose modification is that a ribose 2-site hydroxyl group is substituted by a halogen group or an O-alkyl group.

11. The polymeric nucleic acid molecule as claimed in claim 10, wherein the alkyl is a methyl, an ethyl, a propyl or a methylethyl.

12. The polymeric nucleic acid molecule as claimed in claim 10, wherein the ribose 2-site hydroxyl groups of 5-9 continuous nucleotides, from the first nucleotide at the 3'-end, of the linker segment III of the X-type nucleic acid molecule are substituted by the halogen group or the O-alkyl group;
or, the ribose 2-site hydroxyl groups of 5-9 continuous nucleotides, from the first nucleotide at the 3'-end, of the Hx segment of the H-type nucleic acid molecule are substituted by the halogen group or the O-alkyl group;
or, the ribose 2-site hydroxyl groups of 8-30 continuous nucleotides, from the first nucleotide at the 3'-end, of the H-type nucleic acid molecule are substituted by the halogen group or the O-alkyl group;
or, the ribose 2-site hydroxyl groups of 2-6 continuous nucleotides, from the first nucleotide at the 5'-end, of each segment in the C-type nucleic acid molecule complementary-paired with the targeting segment II of the X-type nucleic acid molecule are substituted by the halogen group or the O-alkyl group.

13. The polymeric nucleic acid molecule as claimed in claim 12, wherein the ribose 2-site hydroxyl groups of 14-18 continuous nucleotides, from the first nucleotide at the 3'-end, of the H-type nucleic acid molecule are substituted by the halogen group or the O-alkyl group.

14. The polymeric nucleic acid molecule as claimed in claim 1, wherein the n is 3 or 4 or 5 or 6 or 7 or 8.

15. The polymeric nucleic acid molecule as claimed in claim 14, wherein the n is 4 or 5 or 6.

16. The polymeric nucleic acid molecule as claimed in claim 1, wherein the number of the target gene is one or two or more, and the number of the target gene does not exceed n;
or, the number of the target gene is 1 or 4 or 6.

17. The polymeric nucleic acid molecule as claimed in claim 1, wherein the target gene is at least one of the following genes: *PPIB, p65, BIRC5, CTNNB, COPS5, CLU, EIF4E, HIF1A, TP53, VEGFA* and *SOD1.*

18. The polymeric nucleic acid molecule as claimed in claim 17, wherein
the polymeric nucleic acid molecule for interfering with an expression of the target gene *PPIB* is the following a1)-a5):
a1) is formed by single-stranded RNA molecules shown in SEQ ID NO:1, SEQ ID NO:2, SEQ ID NO:3 and SEQ ID NO:4;
a2) is formed by single-stranded RNA molecules shown in SEQ ID NO:1, SEQ ID NO:2, SEQ ID NO:3, SEQ ID NO:4, SEQ ID NO:20 and SEQ ID NO:21;
a3) is formed by single-stranded RNA molecules shown in SEQ ID NO:1, SEQ ID NO:2, SEQ ID NO:3, SEQ ID NO:4 and SEQ ID NO:8;
a4) is formed by single-stranded RNA molecules shown in SEQ ID NO:2, SEQ ID NO:3, SEQ ID NO:9, SEQ ID N0:10, SEQ ID NO:11 and SEQ ID NO:12;
a5) is formed by single-stranded RNA molecules shown in SEQ ID NO:2, SEQ ID NO:3, SEQ ID NO:6, SEQ ID NO:9, SEQ ID N0:10, SEQ ID NO:11, SEQ ID NO:13 and SEQ ID NO:14;
or, the polymeric nucleic acid molecule for interfering with an expression of the target gene *P65* is the following b1)-b5):
b1) is formed by single-stranded RNA molecules shown in SEQ ID NO:15, SEQ ID NO:16, SEQ ID NO:17 and SEQ ID NO:18;
b2) is formed by single-stranded RNA molecules shown in SEQ ID NO:6, SEQ ID NO:7, SEQ ID NO:15, SEQ ID NO:16, SEQ ID NO:17 and SEQ ID NO:19;
b3) is formed by single-stranded RNA molecules shown in SEQ ID NO:15, SEQ ID NO:16, SEQ ID NO:17, SEQ ID NO:18 and SEQ ID NO:22;
b4) is formed by single-stranded RNA molecules shown in SEQ ID NO:15, SEQ ID NO:16, SEQ ID NO:17, SEQ ID NO:23, SEQ ID NO:24 and SEQ ID NO:25;
b5) is formed by single-stranded RNA molecules shown in SEQ ID NO:15, SEQ ID NO:16, SEQ ID NO:17, SEQ ID NO:20, SEQ ID NO:23, SEQ ID NO:24, SEQ ID NO:26 and SEQ ID NO:27;
or, the polymeric nucleic acid molecule for simultaneously interfering with expressions of the target genes *BIRC5, CTNNB, COPS5* and *CLU* is formed by single-stranded RNA molecules shown in SEQ ID NO:28, SEQ ID NO:29, SEQ ID NO:30 and SEQ ID NO:31;
or, the polymeric nucleic acid molecule for simultaneously interfering with expressions of the target genes *BIRC5, CTNNB, COPS5, CLU, EIF4E* and *HIF1A* is formed by single-stranded RNA molecules shown in SEQ ID NO:28, SEQ ID NO:29, SEQ ID NO:30, SEQ ID NO:32, SEQ ID NO:33 and SEQ ID NO:34;
or, the polymeric nucleic acid molecule for simultaneously interfering with expressions of the target genes *SOD1, PPIB, P65* and *VEGFA* is the following c1)-c3):
c1) is formed by single-stranded RNA molecules shown in SEQ ID NO:35, SEQ ID NO:36, SEQ ID NO:37 and SEQ ID NO:38;
c2) is formed by single-stranded RNA molecules shown in SEQ ID NO:35, SEQ ID NO:36, SEQ ID NO:37, SEQ ID NO:38, SEQ ID NO:39 and SEQ ID NO:40;
c3) is formed by single-stranded RNA molecules shown in SEQ ID NO:35, SEQ ID NO:36, SEQ ID NO:37, SEQ ID NO:38 and SEQ ID NO:41;
or, the polymeric nucleic acid molecule for interfering with an expression of the target gene *VEGFA* is the following d1)-d11):
d1) is formed by single-stranded RNA molecules shown in SEQ ID NO:42, SEQ ID NO:43, SEQ ID NO:44, SEQ ID NO:45, sequence46 and SEQ ID NO:47;
d2) is formed by single-stranded RNA molecules shown in SEQ ID NO:48, SEQ ID NO:49, SEQ ID NO:50, SEQ ID NO:51, SEQ ID NO:52 and SEQ ID NO:53;
d3) is formed by single-stranded RNA molecules shown in SEQ ID NO:54, SEQ ID NO:55, SEQ ID NO:56, SEQ ID NO:57, SEQ ID NO:58 and SEQ ID NO:59;
d4) is formed by single-stranded RNA molecules shown in SEQ ID NO:42, SEQ ID NO:43, SEQ ID NO:45, SEQ ID NO:46 and SEQ ID NO:47;
d5) is formed by single-stranded RNA molecules shown in SEQ ID NO:48, SEQ ID NO:49, SEQ ID NO:51, SEQ ID NO:52 and SEQ ID NO:53;
d6) is formed by single-stranded RNA molecules shown in SEQ ID NO:54, SEQ ID NO:55, SEQ ID NO:57, SEQ ID NO:58 and SEQ ID NO:59;
d7) is formed by single-stranded RNA molecules shown in SEQ ID NO:42, SEQ ID NO:45, SEQ ID NO:46 and SEQ ID NO:47;
d8) is formed by single-stranded RNA molecules shown in SEQ ID NO:48, SEQ ID NO:51, SEQ ID NO:52 and SEQ ID NO:53;
d9) is formed by single-stranded RNA molecules shown in SEQ ID NO:54, SEQ ID NO:57, SEQ ID NO:58 and SEQ ID NO:59;
d10) is formed by single-stranded RNA molecules shown in SEQ ID NO:60, SEQ ID NO:61, SEQ ID NO:62, SEQ ID NO:63, SEQ ID NO:64 and SEQ ID NO:65;
d11) is formed by single-stranded RNA molecules shown in SEQ ID NO:66, SEQ ID NO:67, SEQ ID NO:68, SEQ ID NO:69, SEQ ID NO:70 and SEQ ID NO:71;
or, the polymeric nucleic acid molecule for interfering with an expression of the target gene *TP53* is the following e1)-e11):
e1) is formed by single-stranded RNA molecules shown in SEQ ID NO:72, SEQ ID NO:73, SEQ ID NO:74, SEQ ID NO:75, SEQ ID NO:76 and SEQ ID NO:77;
e2) is formed by single-stranded RNA molecules shown in SEQ ID NO:78, SEQ ID NO:79, SEQ ID NO:80, SEQ ID NO:81, SEQ ID NO:82 and SEQ ID NO:83;
e3) is formed by single-stranded RNA molecules shown in SEQ ID NO:84, SEQ ID NO:85, SEQ ID NO:86, SEQ ID NO:87, SEQ ID NO:88 and SEQ ID NO:89;
e4) is formed by single-stranded RNA molecules shown in SEQ ID NO:73, SEQ ID NO:74, SEQ ID NO:75, SEQ ID NO:76 and SEQ ID NO:77;
e5) is formed by single-stranded RNA molecules shown in SEQ ID NO:79, SEQ ID NO:80, SEQ ID NO:81, SEQ ID NO:82 and SEQ ID NO:83;
e6) is formed by single-stranded RNA molecules shown in SEQ ID NO:54, SEQ ID NO:55, SEQ ID NO:57, SEQ ID NO:58 and SEQ ID NO:59;
e7) is formed by single-stranded RNA molecules shown in SEQ ID NO:74, SEQ ID NO:75, SEQ ID NO:76 and SEQ ID NO:77;
e8) is formed by single-stranded RNA molecules shown in SEQ ID NO:80, SEQ ID NO:81, SEQ ID NO:82 and SEQ ID NO:83;
e9) is formed by single-stranded RNA molecules shown in SEQ ID NO:86, SEQ ID NO:87, SEQ ID NO:88 and SEQ ID NO:89;
e10) is formed by single-stranded RNA molecules shown in SEQ ID NO:90, SEQ ID NO:91, SEQ ID NO:92, SEQ ID NO:93, SEQ ID NO:94 and SEQ ID NO:95; and
e11) is formed by single-stranded RNA molecules shown in SEQ ID NO:96, SEQ ID NO:97, SEQ ID NO:98, SEQ ID NO:99, SEQ ID NO:100 and SEQ ID NO:101.

19. A derivative of the polymeric nucleic acid molecule as claimed in any one of claims 1-18 is any one of the following (m1)-(m5):
(m1) the polymeric nucleic acid molecule as claimed in any one of claims 1-18 is deleted or added one or more nucleotides, to obtain a derivative of the polymeric nucleic acid molecule having the same function as the polymeric nucleic acid molecule;
(m2) the polymeric nucleic acid molecule as claimed in any one of claims 1-18 is performed nucleotide substitution or modification, to obtain a derivative of the polymeric nucleic acid molecule having the same function as the polymeric nucleic acid molecule;
(m3) a backbone of the polymeric nucleic acid molecule as claimed in any one of claims 1-18 is transformed into a phosphorothioate backbone, to obtain a derivative of the polymeric nucleic acid molecule having the same function as the polymeric nucleic acid molecule;
(m4) a peptide nucleic acid, a locked nucleic acid or an unlocked nucleic acid coded by the polymeric nucleic acid molecule as claimed in any one of claims 1-18 is used, to obtain a derivative of the polymeric nucleic acid molecule having the same function as the polymeric nucleic acid molecule; and
(m5) one end or middle of the polymeric nucleic acid molecule as claimed in any one of claims 1-18 is linked with a signal molecule and/or an active molecule and/or a functional group, to obtain a derivative of the polymeric nucleic acid molecule having the same function as the polymeric nucleic acid molecule.

20. A preparation method for the polymeric nucleic acid molecule as claimed in anyone of claims 1-18, comprising the following steps:
M1) synthesizing the X-type nucleic acid molecule and/or the H-type nucleic acid molecule and/or the C-type nucleic acid molecule as claimed in any one of claims 1-18; and
M2) annealing the X-type nucleic acid molecule and/or the H-type nucleic acid molecule and/or the C-type nucleic acid molecule, to obtain the polymeric nucleic acid molecule.

21. An application of the polymeric nucleic acid molecule as claimed in any one of claims 1-18 or the derivative as claimed in claim 19 in the following A1) or A2):
A1) controlling a target gene expression level in a cell;
A2) preparing a product for preventing and/or relieving and/or treating a disease caused by the target gene expression.

22. The application as claimed in claim 21, wherein the controlling is inhibition or reduction or interference.

23. The application as claimed in claim 21, wherein the cell is a tumor cell.

24. The application as claimed in claim 21, wherein the target gene is a disease-related gene;
or, the disease-related gene is a tumor-related gene;
or, the tumor-related gene is at least one of the following genes: *PPIB, p65, BIRC5, CTNNB, COPS5, CLU, EIF4E, HIF1A, TP53, VEGFA* and *SOD1.*

25. A reagent or a kit or a drug for inhibiting or reducing or interfering with a target gene expression level in a cell, comprising the polymeric nucleic acid molecule as claimed in any one of claims 1-18 or the derivative as claimed in claim 19.

26. A method for inhibiting or reducing or interfering with a target gene expression level in a cell, comprising the following steps:
the polymeric nucleic acid molecule as claimed in any one of claims 1-18 or the derivative as claimed in claim 19 is introduced into the cell, and the expression level of the target gene in the cell is inhibited or reduced.
